# EUROPEAN PATENT SPECIFICATION

(11) **EP 1 749 004 B1**
(45) Date of publication and mention of the grant of the patent: **19.09.2007**
(21) Application number: 05733676.0
(22) Date of filing: 22.04.2005
(51) Int. Cl.: C07D 471/04, A61K 31/519, A61P 29/00

(54) **PYRROLYL SUBSTITUTED PYRIDO[2,3-D]PYRIMIDIN-7-ONES AND DERIVATIVES THEREOF AS THERAPEUTIC AGENTS**
PYRROLYLSUBSTITUIERTE PYRIDO[2,3-D]PYRIMIDIN-7-ONE UND DERIVATE DAVON ALS THERAPEUTISCHE MITTEL
PYRID[2,3-D]PYRIMIDIN-7-ONES PYRROLYL-SUBSTITUEES ET DERIVES DE CES DERNIERES UTILISES COMME AGENTS THERAPEUTIQUES

(30) Priority: 04.05.2004 US 567902 P
(43) Date of publication of application: 07.02.2007
(73) Proprietor: Warner-Lambert Company LLC, New York, NY 10017 (US)
(72) Inventor: BRUENDL, Michelle M., Pfizer Global R&D, Ann Arbor, MI 48105 (US); GOGLIOTTI, Rocco D.., Pfizer Global R&D, Ann Arbor, MI 48105 (US); GOODMAN, A.P.., Pfizer Global R&D, Ann Arbor, MI 48105 (US); REICHARD, Gregory., Pfizer Global R&D, Ann Arbor, MI 48105 (US)
(74) Representative: Nevant, Marc
(86) International application number: PCT/IB2005/001141
(87) International publication number: WO 2005/105801

(56) References cited:
- EP-A- 1 277 738
- US-A1- 2004 009 993

## Description

### BACKGROUND OF THE INVENTION

Phosphoinositide-3-kinases (PI3Ks) are a family of lipid kinases that phosphorylate phosphoinositols on the 3'-OH to generate PI-3-P (phosphatidylinositol 3-phosphate), PI-3,4-P2 and PI-3,4,5-P3. One class of PI3Ks are stimulated by growth factors. A separate class of PI3Ks are activated by G-protein coupled receptors and include PI3Kγ. The growth-factor stimulated PI3Ks (e.g., PI3Kα), have been implicated in cellular proliferation and cancer. PI3Kγ has been demonstrated to be involved in signaling cascades. For example, PI3Kγ is activated in response to ligands such as C5a, fMLP, ADP, and IL-8. In addition, PI3Kγ has been implicated in immune diseases (Hirsch et al. Science 2000;287:1049-1053). PI3Kγ null macrophages show a reduced chemotactic response and a reduced ability to fight inflammation (Hirsch et al., 2000, supra). Furthermore, PI3Kγ has also been implicated in thrombolytic diseases (e.g., thromboembolism, ischemic diseases, heart attacks, and stroke) (Hirsch et al. FASEB J. 2000;15(11):2019-2021; and Hirsch et al. FASEB J., July 9 2001;10.1096/fj.00-0810fje (cited herein as Hirsch et al., 2001).

PI3K inhibitors are being pursued for the treatment of human disease (see e.g., WO 01/81346; WO 01/53266; WO 01/83456; and WO 2004/007491). There is a need for additional compounds that can inhibit PI3Ks for use as pharmaceutical agents.

### SUMMARY OF THE INVENTION

In one aspect, the present invention provides for pyridopyrimidines of formula I: 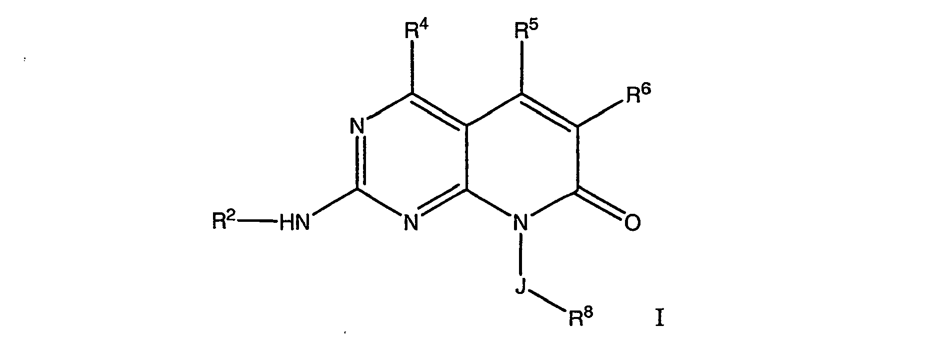
or a pharmaceutically acceptable salt thereof;
R⁴ is selected from the group consisting of:
   methyl, ethyl, CF₃, CH₂F, CHF₂, and CH₂OH;
   R⁵ is H or methyl;
   J is absent or a C₁-C₆ alkylene;
   R⁸ is selected from the group consisting of: a C₁-C₆ alkyl, a C₃-C₈ cycloalkyl, a 5- or 6-membered heterocycloalkyl, a 5- or 6- membered heteroaryl, and a phenyl;
wherein R⁶ is selected from the group consisting of: H, halo, a phenyl, and a C₁-C₃ alkyl;
wherein R² is: 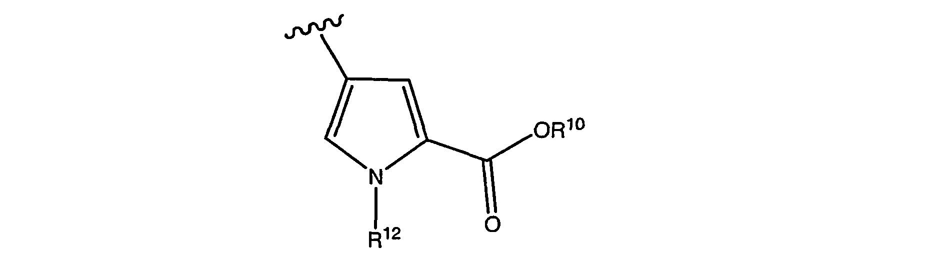 or 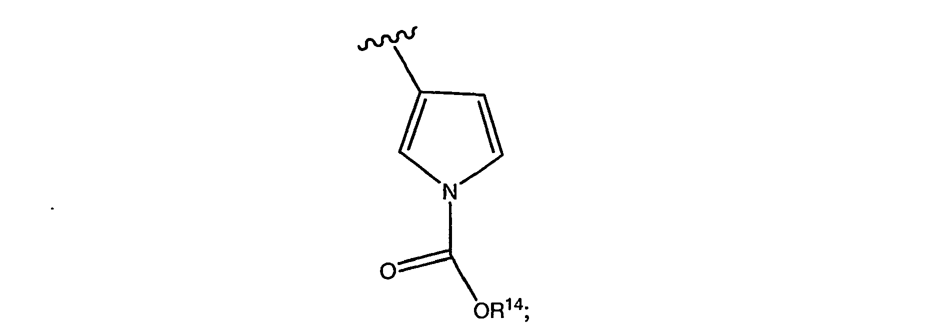
   R¹⁰ is a C₁-C₆ alkyl or H;
   R¹² is a C₁-C₆ alkyl, or a C₁-C₃alkylene-R¹³,
   R¹³ is a pyridinyl or a phenyl; and
   R¹⁴ is a C₁-C₆ alkyl or H.

In certain embodiments of Formula I, R⁴ is a methyl, R⁵ is H, and R² is: 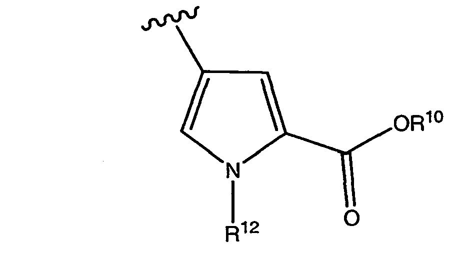 - a compound of Formula II:

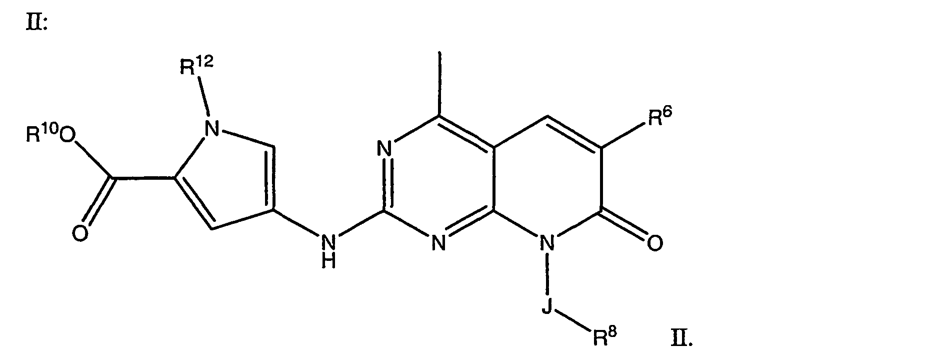

In certain embodiments of Formula II, R¹² is a C₁-C₃alkylene-R¹³. In certain embodiments of Formula II, R⁸ is a C₁-C₆ alkyl. An example of a compound of Formula II where R⁸ is a C₁-C₆ alkyl is: 4-(4,8-Dimethyl-7-oxo-7,8-dihydro-pyrido[2,3-d]pyrimidin-2-ylamino)-1-(1-phenyl-ethyl)-1H-pyrrole-2-carboxylic acid. In other embodiments of Formula II, R⁸ is selected from the group consisting of: a C₃-C₈ cycloalkyl, a 5- or 6-membered heterocycloalkyl, a 5- or 6- membered heteroaryl, and phenyl. Examples of a compound of Formula II where R⁸ is selected from the group consisting of: a C₃-C₈ cycloalkyl, a 5- or 6- membered heterocycloalkyl, a 5- or 6-membered heteroaryl, and phenyl include, but are not limited to:
4-(8-Cyclopentyl-6-fluoro-4-methyl-7-oxo-7,8-dihydro-pyrido[2,3-d]pyrimidin-2-ylamino)-1-pyridin-3-ylmethyl-1H-pyrrole-2-carboxylic acid ethyl ester;
4-(8-Cyclopentyl-6-fluoro-4-methyl-7-oxo-7,8-dihydro-pyrido[2,3-d]pyrimidin-2-ylamino)-1-(1-phenyl-ethyl)-1H-pyrrole-2-carboxylic acid; and
1-Benzyl-4-(8-cycloheptyl-4-methyl-7-oxo-7,8-dihydro-pyrido[2,3-d]pyrimidin-2-ylamino)-1H-pyrrole-2-carboxylic acid ethyl ester.

In other embodiments of Formula II, R¹² is a C₁-C₆ alkyl and R⁸ is a C₁-C₆ alkyl, a C₃-C₈ cycloalkyl, a 5- or 6-membered heterocycloalkyl, a 5- or 6- membered heteroaryl, or a phenyl. Examples of such compounds include, but are not limited to:
4-(6-Chloro-8-ethyl-4-methyl-7-oxo-7,8-dihydro-pyrido[2,3-d]pyrimidin-2-ylamino)-1-methyl-1H-pyrrole-2-carboxylic acid methyl ester;
4-[6-Bromo-8-(2-methoxy-ethyl)-4-methyl-7-oxo-7,8-dihydro-pyrido[2,3-d]pyrimidin-2-ylamino]-1-methyl-1H-pyrrole-2-carboxylic acid;
4-(6-Chloro-8-ethyl-4-methyl-7-oxo-7,8-dihydro-pyrido[2,3-d]pyrimidin-2-ylamino)-1-methyl-1H-pyrrole-2-carboxylic acid.
4-(8-Cyclohexyl-4-methyl-7-oxo-7,8-dihydro-pyrido[2,3-d]pyrimidin-2-ylamino)-1-methyl-1H-pyrrole-2-carboxylic acid methyl ester;
4-[6-Fluoro-8-(4-methoxy-cyclohexyl)-4-methyl-7-oxo-7,8-dihydro-pyrido[2,3-d]pyrimidin-2-ylamino]-1-methyl-1H-pyrrole-2-carboxylic acid methyl ester;
4-[6-Fluoro-4-methyl-7-oxo-8-(tetrahydro-pyran-4-ylmethyl)-7,8-dihydro-pyrido[2,3-d]pyrimidin-2-ylamino]-1-methyl-1H-pyrrole-2-carboxylic acid methyl ester;
4-(8-Cyclopentyl-6-fluoro-4-methyl-7-oxo-7,8-dihydro-pyrido[2,3-d]pyrimidin-2-ylamino)-1-methyl-1H-pyrrole-2-carboxylic acid;
4-(8-Cyclopentyl-6-fluoro-4-methyl-7-oxo-7,8-dihydro-pyrido[2,3-d]pyrimidin-2-ylamino)-1-methyl-1H-pyrrole-2-carboxylic acid methyl ester;
4-[8-(2-Cyclopropyl-ethyl)-4-methyl-7-oxo-7,8-dihydro-pyrido[2,3-d]pyrimidin-2-ylamino]-1-methyl-1H-pyrrole-2-carboxylic acid methyl ester;
4-(8-Cyclobutyl-4-methyl-7-oxo-7,8-dihydro-pyrido[2,3-d]pyrimidin-2-ylamino)-1-methyl-1H-pyrrole-2-carboxylic acid methyl ester;
4-[6-Bromo-8-(4-methoxy-benzyl)-4-methyl-7-oxo-7,8-dihydro-pyrido[2,3-d]pyrimidin-2-ylamino]-1-methyl-1H-pyrrole-2-carboxylic acid;
4-(8-Cyclopropyl-4-methyl-7-oxo-7,8-dihydro-pyrido[2,3-d]pyrimidin-2-ylamino)-1-methyl-1H-pyrrole-2-carboxylic acid methyl ester;
4-[6-Fluoro-8-(4-methoxy-cyclohexyl)-4-methyl-7-oxo-7,8-dihydro-pyrido[2,3-d]pyrimidin-2-ylamino]-1-methyl-1H-pyrrole-2-carboxylic acid; and
4-[8-Cyclohexyl-4-methyl-7-oxo-7,8-dihydro-pyrido[2,3-d]pyrimidin-2-ylamino]-1-methyl-1H-pyrrole-2-carboxylic acid.

In certain embodiments of Formula I, R⁶ is methyl, and R⁴ is a C₃-C₈ cycloalkyl, and R² is: 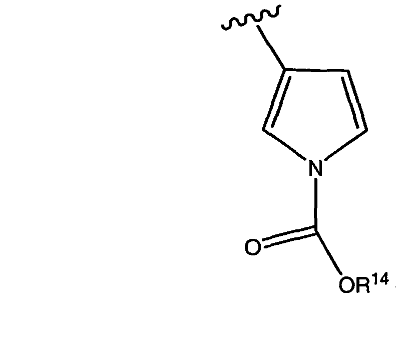 - a compound of Formula III:

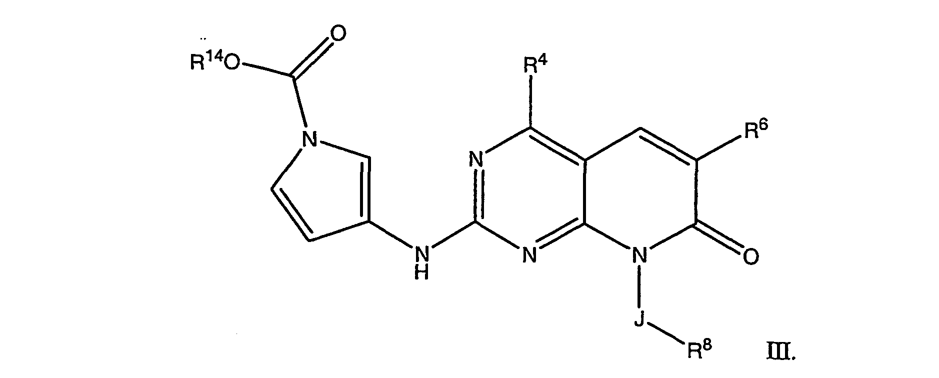

In certain embodiments of Formula III, R⁸ is a C₁-C₆ alkyl, a C₃-C₈ cycloalkyl, a 5- or 6-membered heterocycloalkyl, a 5- or 6-membered heteroaryl, or a phenyl.

In certain embodiments of Formulas I, II, or III, R⁶ is halo. In other embodiments of of Formulas I, II, or III, R⁶ is H. In still other embodiments of Formulas I, II, or III, R⁶ is phenyl. In yet still other embodiments of Formulas I, II, or III, R⁶ is a C₁-C₃ alkyl.

In another aspect, the invention provides for pharmaceutical compositions that comprise a therapeutically effective amount of a compound of any one of Formulas I-III and a pharmaceutically acceptable carrier. In certain embodiments, these compositions are useful in the treatment of a PI3K-mediated disease. The compounds of the invention can also be combined in a pharmaceutical composition that also comprise compounds that are useful for the treatment of cancer, a thrombolytic disease, heart disease, stroke, an inflammatory disease such as rheumatoid arthritis, or another PI3K-mediated disease.

In certain embodiments, the PI3K-mediated disease is selected from the group consisting of: rheumatoid arthritis, ankylosing spondylitis, osteoarthritis, psoriatic arthritis, psoriasis, inflammatory diseases, and autoimmune diseases. In other embodiments, the PI3K-mediated disease is selected from the group consisting of: cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, and coronary artery disease. In still other embodiments, the PI3K-mediated disease is selected from the group consisting of: cancer, colon cancer, glioblastoma, endometrial carcinoma, hepatocellular cancer, lung cancer, melanoma, renal cell carcinoma, thyroid carcinoma, cell lymphoma, lymphoproliferative disorders, small cell lung cancer, squamous cell lung carcinoma, glioma, breast cancer, prostate cancer, ovarian cancer, cervical cancer, and leukemia. In yet another embodiment, the PI3K-mediated disease is selected from the group consisting of: type II diabetes. In still other embodiments, the PI3K-mediated disease is selected from the group consisting of: respiratory diseases, bronchitis, asthma, and chronic obstructive pulmonary disease. In certain embodiments, the subject is a human.

In another aspect, the present invention provides for the use of a compound of Formula I in the manufacture of a medicament for the treatment of a disorder in mammals, wherein the disorder is selected from cancer, breast cancer, gliobastoma, endometrial carcinoma, heptocellular carcinoma, colon cancer, lung cancer, melanoma, renal cell carcinoma, thyroid carcinoma, small cell lung cancer, squamous cell lung carcinoma, glioma, breast cancer, prostate cancer, ovarian cancer, cervical cancer, leukemia, cell lymphoma, lymphoproliferative disorders. The present invention also provides for the use of a compound of of Formula I in the manufacture of a medicament for the treatment of rheumatoid arthritis in mammals, as well as the the use of a compound of of Formula I in the manufacture of a medicament for the treatment of chronic obstructive pulmonary disease in mammals.

### DEFINITIONS

A "PI3K-mediated disease" is characterized by the participation of one or more PI3Ks or a PI3P phosphatase, (e.g., PTEN (Phosphatase and Tensin homolog deleted on chromosome Ten), etc.) in the inception, manifestation of one or more symptoms or disease markers, severity, or progression of a disease. PI3K-mediated diseases include, but are not limited to: rheumatoid arthritis, ankylosing spondylitis, osteoarthritis, psoriatic arthritis, psoriasis, inflammatory diseases, pulmonary fibrosis, autoimmune diseases, cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, coronary artery disease, cancer, breast cancer, gliobastoma, endometrial carcinoma, hepatocellular carcinoma, colon cancer, lung cancer, melanoma, renal cell carcinoma, thyroid carcinoma, small cell lung cancer, squamous cell lung carcinoma, glioma, prostate cancer, ovarian cancer, cervical cancer, leukemia, cell lymphoma, lymphoproliferative disorders, type II diabetes, respiratory diseases, bronchitis, asthma, and chronic obstructive pulmonary disease.

A PI3K is an enzyme that is able to phosphorylate the 3'-OH of a phosphoinositol to generate PI3P. PI3Ks include, but are not limited to, PI3Kα, PI3Kβ, PI3Kγ, and PI3Kδ. A PI3K typically comprises at least one catalytic subunit (e.g., p110γ), and may further comprise a regulatory subunit (e.g., p101, etc.).

The term "alkyl group" or "alkyl" includes straight and branched carbon chain radicals. The term "alkylene" refers to a diradical of an unsubstituted or substituted alkane. For example, a "C₁₋₆ alkyl" is an alkyl group having from 1 to 6 carbon atoms. Examples of C₁-C₆ straight-chain alkyl groups include, but are not limited to, methyl, ethyl, n-propyl, n-butyl, n-pentyl, and n-hexyl. Examples of branched-chain alkyl groups include, but are not limited to, isopropyl, *tert*-butyl, isobutyl, etc. Examples of alkylene groups include, but are not limited to, -CH₂-, -CH₂-CH₂-, -CH₂-CH(CH₃)-CH₂-, and -(CH₂)₁₋₃. Alkylene groups can be substituted with groups as set forth below for alkyl.

The term alkyl includes both "unsubstituted alkyls" and "substituted alkyls," the latter of which refers to alkyl moieties having substituents replacing a hydrogen on one or more carbons of the hydrocarbon backbone. Such substituents are independently selected from the group consisting of: halo, I, Br, Cl, F, -OH, -COOH, trifluoromethyl, -NH₂, -OCF₃, and O-C₁-C₃ alkyl.

Typical substituted alkyl groups thus are 2,3-dichloropentyl, 3-hydroxy-5-carboxyhexyl, 2-aminopropyl, pentachlorobutyl, trifluoromethyl, methoxyethyl, 3-hydroxypentyl, 4-chlorobutyl, 1,2-dimethyl-propyl, and pentafluoroethyl.

"Halo" includes fluoro, chloro, bromo, and iodo.

The term "C₃-C₈cycloalkyl" refers to a cycloalkyl group containing from 3 to 8 carbons. Thus, the term "C₃-C₈cycloalkyl" encompasses monocyclic cycloalkyl groups containing from 3 to 8 carbons and bicyclic cycloalkyl groups containing 7 or 8 carbons. Examples of "C₃-C₈cycloalkyls" include, but are not limited to, cyclopropyl, cyclobutyl, cyclopentyl, cyclohexyl, cycloheptyl, and bicyclo[2.2.1]heptyl; the cycloalkyl group may optionally contain 1 or 2 double bonds (i.e., a cycloalkylenyl) including, but not limited to, cyclopentenyl, cyclohexenyl, and cycloheptenyl. A "C₃-C₈cycloalkyl" may be substituted with 1 or 2 groups independently selected from -OH, C₁-C₄alkyl (e.g., methyl) and -O-C₁-C₄alkyl (e.g., methoxy). Examples of substituted cycloalkyl groups include, but are not limited to, methyl-cyclopropyl, dimethyl-cyclohexyl, 2-methyl-cyclohexyl, 3-methyl-cyclohexyl, 3,5-dimethyl-cyclohexyl, and 4-methyl-cyclohexyl.

A "5-membered heterocycloalkyl" is a stable 5-membered, monocyclic cycloalkyl ring having from 2 to 4 carbon atoms and from 1 to 3 heteroatoms selected from the group consisting of: 1 O; 1 S; 1 N; 2 N; 1 S and 1 N; 1 S, and 2 N; 1 O and 1 N; and 1 O and 2 N, wherein when two O atoms or one O atom and one S atom are present in a ring, the two O atoms or one O atom and one S atom are not bonded directly to each other, respectively. Illustrative examples of stable 5-membered heterocycloalkyls include tetrahydrofuranyl, dihydrofuranyl, tetrahydrothienyl, dihydrothienyl, imidazolidinyl, oxazolidinyl, imidazolinyl, isoxazolidinyl, pyrrolidinyl, 2-pyrrolinyl, and 3-pyrrolinyl.

A "6-membered heterocycloalkyl" is a stable 6-membered, monocyclic cycloalkyl ring having from 3 to 5 carbon atoms and from 1 to 3 heteroatoms selected from the group consisting of: 1 O; 2 O;1 S; 2 S; 1 N; 2 N; 3 N; 1 S, 1 O, and 1 N; 1 S and 1 N; 1 S and 2 N; 1 S and 1 O; 1 S and 2 O; 1 O and 1 N; and 1 O and 2 N, wherein when two O atoms or one O atom and one S atom are present, the two O atoms or one O atom and one S atom are not bonded directly to each other, respectively. Illustrative examples of stable 6-membered heterocycloalkyls include tetrahydropyranyl, dihydropyranyl, dioxanyl, 1,3-dioxolanyl, 1,4-dithianyl, hexahydropyrimidine, morpholinyl, piperazinyl, piperidinyl, 2H-pyranyl, 4H-pyranyl, pyrazolidinyl, pyrazolinyl, 1,2,3,6-tetrahydropyridinyl, tetrahydrothiopyranyl, 1,1-dioxo-hexahydro-1λ⁶-thiopyranyl, 1,1-dioxo-1λ⁶-thiomorpholinyl, thiomorpholinyl, thioxanyl, and 1,3,5-trithianyl.

Embraced within the term "5 or 6 membered heterocycloalkyl" are 5 membered rings having one carbon-carbon or one carbon-nitrogen double bond in the ring (e.g., 2-pyrrolinyl, 3-pyrrolinyl, etc.) and 6 membered rings having one carbon-carbon or one carbon-nitrogen double bond in the ring (e.g., dihydro-2H-pyranyl, 1,2,3,4-tetrahydropyridine, 3,4-dihydro-2H-[1,4]oxazine, etc.).

"5 or 6-membered heterocycloalkyls" may be unsubstituted or substituted with groups such as those set out above for C₃-C₈cycloalkyls, where possible, and with an oxo or thiooxo group on a carbon ring atom.

Unless otherwise indicated, the foregoing heterocycloalkyls can be C-attached or N-attached where such is possible and which results in the creation of a stable structure. For example, piperidinyl can be piperidin-1-yl (N-attached) or piperidin-4-yl (C-attached).

The term "phenyl" refers to unsubstituted and substituted phenyl groups. A phenyl group may be substituted with 4 halo substituents, or with 1 to 3 substituents independently selected from the group consisting of: halo, I, Br, Cl, F, -CH₂OH, -OH, -COOH, trifluoromethyl, -NH₂, -C(O)NH₂, -OCF₃, -O-C₁-C₃ alkyl, C₁-C₃alkyl, -O-C₁-C₃alkyl,-OCF₃, -O-phenyl, -O-C₁-C₃alkylene-phenyl, and a C₅-C₆ cycloalkyl, wherein the -O-C₁-C₃ alkyl, C₁-C₃alkyl, -O-C₁-C₃alkyl, - O-phenyl, and the C₅-C₆ cycloalkyl may be optionally substituted with 1 to 3 substituents selected from halo, benzyl, and -OH.

Typical substituted phenyl groups include, but are not limited to, 3-benzyloxy-phenyl, 3-chlorophenyl, 2,6-dibromophenyl, 2,4,6-tribromophenyl, 2,6-dichlorophenyl, 4-trifluoromethylphenyl, 3-methyl-phenyl, 4-methyl-phenyl, 3,5-dimethyl-phenyl, 3,4,5-trimethoxy-phenyl, 3,5-dimethoxy-phenyl, 3,4-dimethoxy-phenyl, 3-methoxy-phenyl, 4-methoxy-phenyl, 3,5-difluoro-phenyl, 4-chloro-phenyl, 3-trifluoromethyl-phenyl, 3,5-dichloro-phenyl, 2-methoxy-5-methyl-phenyl, 2-fluoro-5-methyl-phenyl, 4-chloro-2-trifluoromethyl-phenyl, and the like.

A "5-membered heteroaryl" is a stable 5-membered, monocyclic, aromatic ring radial having from 1 to 4 carbon atoms and from 1 to 4 heteroatoms selected from the group consisting of: 1 O; 1 S; 1 N; 2 N; 3 N; 4 N; 1 S and 1 N; 1 S and 2 N; 1 O and 1 N; and 1 O and 2 N. Illustrative examples of stable 5-membered heteroaryls include, but are not limited to, furanyl, 2-furanyl, 3-furanyl, imidazolyl, isoxazolyl, isothiazolyl, oxadiazolyl, oxazolyl, pyridinyl, 2-, 3-, or 4-pyridinyl, pyrimidinyl, 2-, 4-, or 5-pyrimidinyl, pyrazolyl, pyrrolyl, 2- or 3-pyrrolyl, pyrazinyl, pyridazinyl, 3- or 4-pyridazinyl, 2-pyrazinyl, thienyl, 2-thienyl, 3-thienyl, tetrazolyl, thiazolyl, thiadiazolyl, triazinyl and triazolyl.

A "6-membered heteroaryl" is a stable 6-membered, monocyclic, aromatic ring radical having from 3 to 5 carbon atoms and from 1 to 3 heteroatoms selected from the group consisting of: 1 N; 2 N; and 3 N. Illustrative examples of stable 6-membered heteroaryl include pyridin-2-yl, pyridin-4-yl, pyrimidin-2-yl, pyridazin-4-yl, and pyrazin-2-yl.

A heteroaryl can also include ring systems substituted on ring carbons with one or more -OH functional groups (which may tautomerize to give a ring C=O group) and/or substituted on a ring sulfur atom by 1 or 2 oxygen atoms to give S=O, or SO₂ groups, respectively.

Carbon ring atoms in a 5 or 6 membered heteroaryl may be optionally substituted where possible with one or two groups selected from NH₂, hydroxy, (C₁-C₆)alkyl, (C₁-C₆)alkyl-SO₂-NH-, (C₁-C₆)alkyl-(C=O)-, (C₁-C₆)alkyl-NH-(C=O)-, and (C₁-C₆)alkyl-SO₂-.

Nitrogen ring atoms in a 5 or 6 membered heteroaryl may be optionally substituted where possible with a H₂N(C=O)-, or with a (C₁-C₆)alkyl, (C₁-C₆)alkyl-(C=O)-, (C₁-C₆)alkyl-NH-(C=O)-, [(C₁-C₆)alkyl]₂-N-(C=O)-, or (C₁-C₆)alkyl-O(C=O)-.

Some of the compounds in the present invention may exist as stereoisomers, including enantiomers, diastereomers, and geometric isomers. Geometric isomers include compounds of the present invention that have alkenyl groups, which may exist as entgegen or zusammen conformations, in which case all geometric forms thereof, both entgegen and zusammen, *cis* and *trans,* and mixtures thereof, are within the scope of the present invention. Some compounds of the present invention have cycloalkyl groups, which may be substituted at more than one carbon atom, in which case all geometric forms thereof, both *cis* and *trans,* and mixtures thereof, are within the scope of the present invention. All of these forms, including (R), (S), epimers, diastereomers, cis, trans, syn, anti, (E), (Z), solvates (including hydrates), tautomers, and mixtures thereof, are contemplated in the compounds of the present invention.

### DETAILED DESCRIPTION OF THE INVENTION

### I. INTRODUCTION

The present invention relates to pyridopyrimidines of Formulas I-III, wherein R², R⁴, R⁵, R⁶, R⁸ and J have any of the values defined therefor in the specification, and pharmaceutically acceptable salts thereof, that are useful as agents in the treatment of diseases and conditions, including inflammatory diseases, cardiovascular diseases, and cancers. Also provided are pharmaceutical compositions comprising one or more compounds of Formulas I-III.

### II. PREPARATION OF COMPOUNDS

Compounds of the present invention (e.g., compounds of Formulas I) can be prepared by applying synthetic methodology known in the art and synthetic methodology outlined in the schemes set forth below.

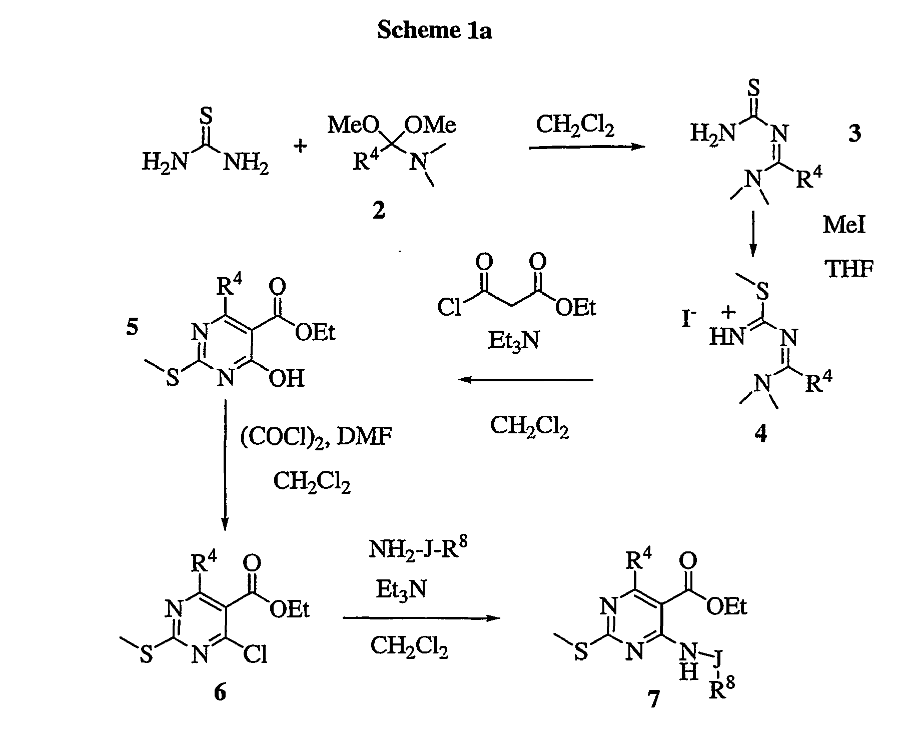

In Scheme 1a, thiourea in dichloromethane is reacted with **2** (e.g., 1,1-dimethoxy-ethyl)-dimethyl-amine) to yield the thiourea **3** (e.g., (1-dimethylamino-ethylidene)-thiourea). **3** is then alkylated with iodomethane (MeI) in THF (tetrahydrofuran) to yield the 2-methyl-isothiourea **4** (e.g., 1-(1-dimethylamino-ethylidene)-2-methyl-isothiourea). The isothiourea **4** in a chlorinated hydrocarbon solvent (e.g., dichloromethane) is treated with chlorocarbonyl-acetic acid ethyl ester, followed by a tertiary amine such as triethylamine to provide the pyrimidine **5** (e.g., 4-hydroxy-6-methyl-2-methylsulfanyl-pyrimidine-5-carboxylic acid ethyl ester).

**5** in dichloromethane is reacted with oxalyl chloride and a catalytic amount of DMF (dimethylformamide) to yield **6** (e.g., 4-Chloro-6-methyl-2-methylsulfanyl-pyrimidine-5-carboxylic acid ethyl ester). The chloro group of **6** is then displaced with an amine (NH₂-J-R⁸) in the presence of triethylamine in a solvent such as dichloromethane to provide **7** (e.g., 4-cycloheptylamino-6-methyl-2-methyl-sulfanyl-pyrimidine-5-carboxylic acid ethyl ester). A variety of amines (NH₂-J-R⁸) can be used including, but not limited to: cycloheptylamine, cyclopentylamine, cyclohexylamine, methylamine, and 2-cyclopropyl-ethylamine.

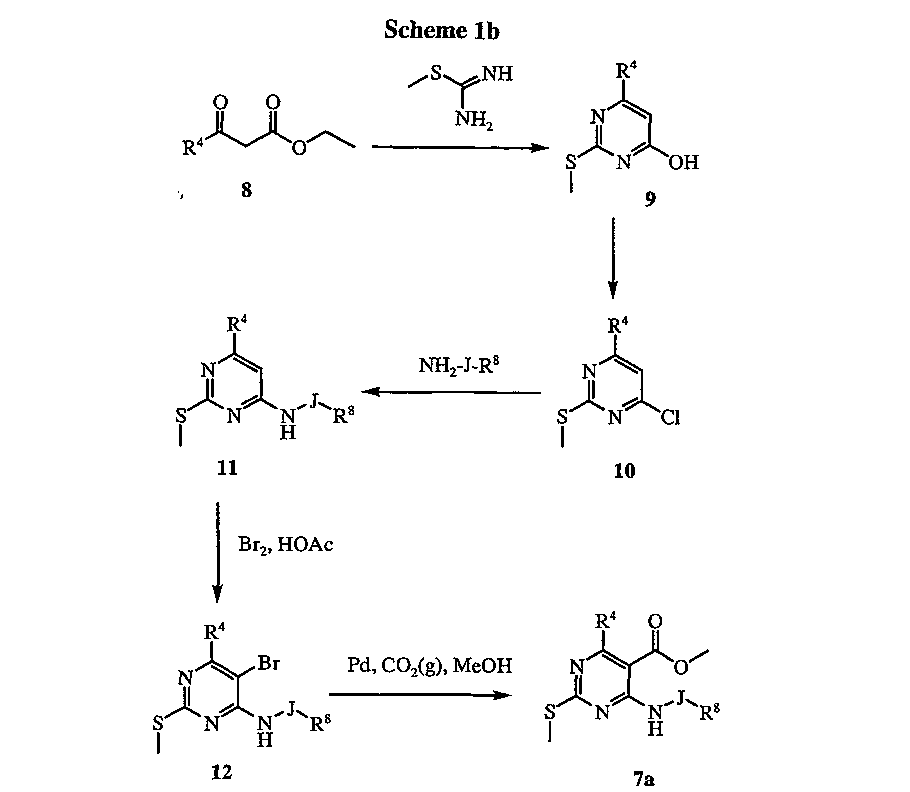

In Scheme 1b, 8 (e.g., 4,4,4-trifluoro-3-oxo-butyric acid ethyl ester) is treated with 2-methyl-isothiourea in a solvent such as dichloromethane to yield **9** (e.g., 2-methylsulfanyl-6-trifluoromethyl-pyrimidin-4-ol). **9** in a solvent such as dichloromethane is then chlorinated with oxalyl chloride and a catalytic amount of DMF to yield **10** (e.g., 4-chloro-2-methylsulfanyl-6-trifluoromethyl-pyrimidine).

The chloro group of **10** is then displaced with an amine, NH₂-J-R⁸, to generate **11** (e.g., methyl-(2-methylsulfanyl-6-trifluoromethyl-pyrimidin-4-yl)-amine). **11** is then brominated with bromine in acetic acid to provide **12** (e.g., (5-bromo-2-methylsulfanyl-6-trifluoromethyl-pyrimidin-4-yl)-methyl-amine). The bromo group of **12** can then be converted to the ester **7a** (e.g., 4-methylamino-2-methylsulfanyl-6-trifluoromethyl-pyrimidine-5-carboxylic acid methyl ester) using a palladium catalyst with CO₂(*g*), in methanol.

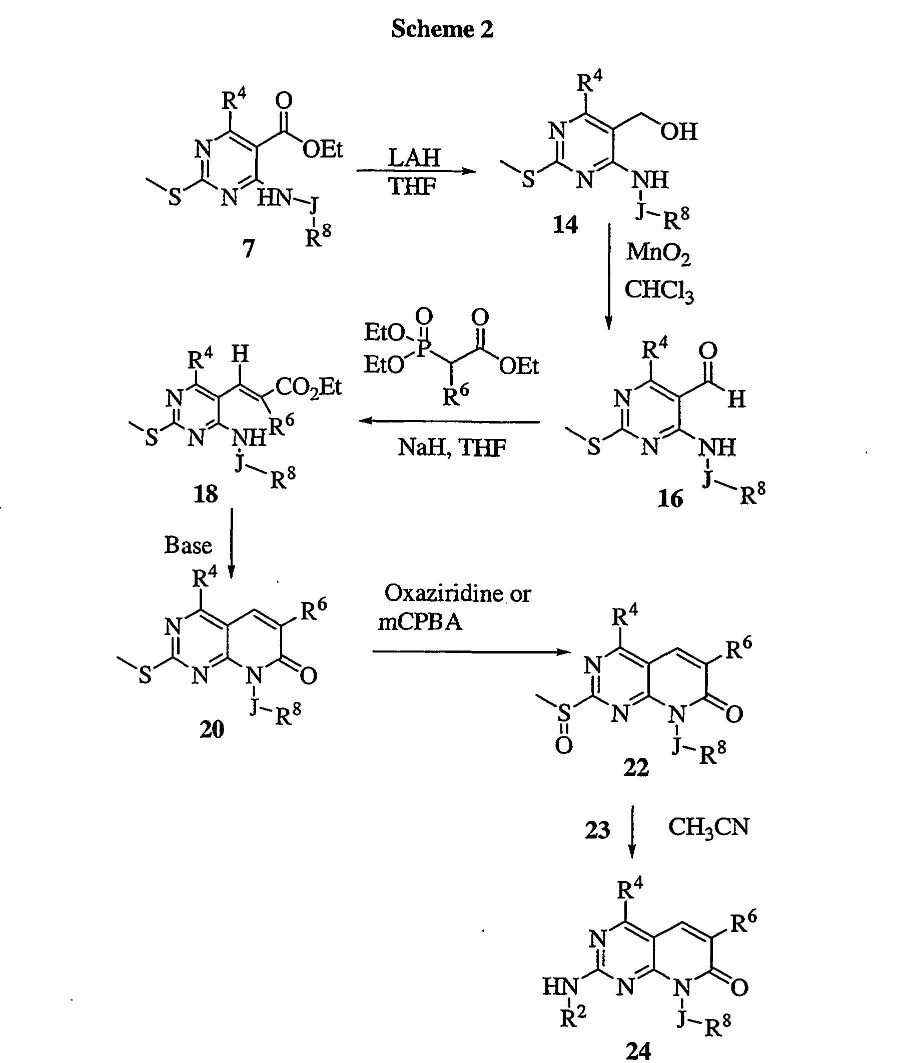

In Scheme 2, 7 (or 7a) in THF can be reduced with a hydride such as lithium aluminum hydride (LAH) to provide the alcohol **14** (e.g., 4-cycloheptylamino-6-methyl-2-methyl-sulfanyl-pyrimidin-5-yl)-methanol). **14** in chloroform can then be oxidized using a metal dioxide such as manganese dioxide (MnO₂) to provide **16** (e.g., 4-cycloheptylamino-6-methyl-2-methyl-sulfanylpyrimidin-5-carbaldehyde).

**16** is then reacted with either a stabilized phosphorane, a phosphonate ester (e.g., (Et-O)₂-P(O)-CH(R⁶)-COOEt, where R⁶ is Br, Cl, F, or H) in the presence of a base, or an alternative suitable Wittig or Horner-Emmons-Wadsworth reagent to provide the corresponding unsaturated ester **18** (e.g., 3-(4-cycloheptylamino-6-methyl-2-methyl-sulfanyl-pyrimidin-5-yl)-acrylic acid ethyl ester). For example, the transformation of **16** to **18** can be carried out in a solvent such as THF with sodium hydride treated ethoxyphosphinoyl-acetic acid ethyl ester or triethyl 2-fluoro-2-phosphonoacetate.

The treatment of **18** in DMF with a tertiary amine such as 1,8-diazabicyclo[5.4.0]undec-7-ene (DBU) or 1,5-diazabicyclo[4.3.0]non-5-ene (DBN), with a catalytic amount of potassium *tert*-butoxide effects ring closure to provide **20** (e.g., 8-cycloheptyl-4-methyl-2-methylsulfanyl-8H-pyrido[2,3-d]pyrimidin-7-one).

The methylthio group of a 2-methylsulfanyl-8H-pyrido[2,3-d]pyrimidin-7-one **8** (e.g., 4-methyl-2-methylsulfanyl-8H-pyrido[2,3-d]pyrimidin-7-one) in dichloromethane or chloroform is then oxidized to the corresponding methyl sulfinyl derivative using a suitable oxaziridine (e.g., Davis oxaziridine ((1S)-(+)-(10-camphorsulfonyl)oxaziridine); or 3-phenyl-2-(phenylsulfonyl)-1,2-oxaziridine, etc.), dimethyldioxirane, or mCPBA (3-chloroperoxybenzoic acid) in a solvent such as chloroform or dichloromethane to provide **22** (e.g., 8-cycloheptyl-4-methyl-2-methylsulfinyl-8H-pyrido[2,3-d]pyrimidin-7-one). The methyl sulfinyl group of **22** is then displaced with an amino-pyrrole **23** (R²-NH₂) (e.g., 4-amino-1-benzyl-1H-pyrrole-2-carboxylic ethyl ester, etc.) in acetonitrile to yield the 8H-pyrido[2,3-d]pyrimidin-7-one **24** (e.g., 1-Benzyl-4-(8-cycloheptyl-4-methyl-7-oxo-7,8-dihydro-py/do[2,3-d]pyrimidin-2-ylamino)-1H-pyrrole-2-carboxylic acid ethyl ester). A variety of R²-NH₂ compounds can be used, including but not limited to: 4-amino-1-methyl-1H-pyrrole-2-carboxylic acid ethyl ester, 4-amino-1-pyridin-3-ylmethyl-1H-pyrrole-2-carboxylic acid ethyl ester, 4-amino-1-(1-phenyl-ethyl)-1H-pyrrole-2-carboxylic acid ethyl ester, 4-amino-1-methyl-1H-pyrrole-2-carboxylic acid methyl ester, and 4-amino-1-isopropyl-1H-pyrrole-2-carboxylic acid ethyl ester. A subsequent deprotection step and/or saponification step may be required depending on the nature of substituents such as R² or R⁸. For example, an ester group can be hydrolyzed to the corresponding acid with a suitable base (e.g., NaOH, LiOH). Scheme 3 depicts an example of a deprotection reaction scheme.

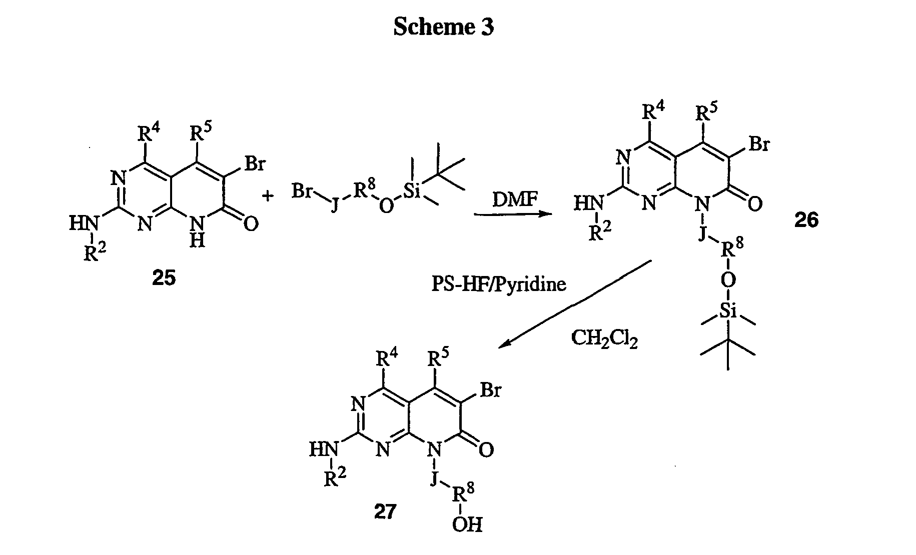

In certain embodiments, protecting groups on moieties such as R² or R⁸ are used during the synthesis of compounds of the present invention. Those of skill in the art will recognize that there are wide variety of protecting groups that can be used in organic syntheses (see e.g., Greene and Wuts, Protective Groups in Organic Synthesis, 2nd ed., (John Wiley & Sons, Inc., 1991)). For example, in Scheme 3, a pyridopyrimidine **25** in DMF is reacted with polystyrene supported BEMP-resin (2-tert-butylimino-2-diethylamino-1,3-dimethyl-perhydro-1,3,2-diazaphosphorine) and a Br-J-R⁸-TBDMS compound (e.g., (3-bromo-propoxy)-*tert*-butyl-dimethyl-silane) to yield **26.** Protecting groups for hydroxyls such as the silyl ether protecting group t-butyl-dimethylsilyl (TBDMS) ether group are known in the art (see e.g., Greene and Wuts, Protective Groups in Organic Synthesis, 2nd ed., (John Wiley & Sons, Inc., 1991)). The silyl ether group of **26** can then be removed by acid hydrolysis such as a hydrogen fluoride-pyridine (35-40% HF) polymer bound resin to provide **27.**

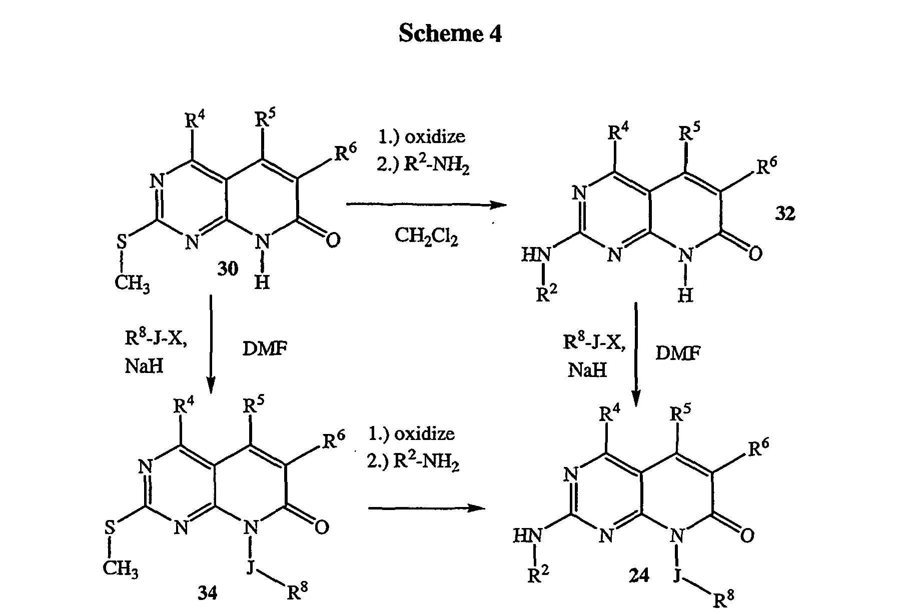

In Scheme 4, the methylsulfanyl group **30** can be oxidized to a methylsulfinyl group using an oxirane such as dimethyldioxirane, in a solvent such as methylene chloride, or an oxaziridine and then reacted with an amine R²-NH₂ in acetonitrile or with DCM (4-(dicyanomethylene)-2-methyl-6-(4-dimethylaminostyryl)-4H-pyran), and PS-morpholine,(polystyrene-morpholine), in a solvent such as DMF to provide **32. 32** can then be reacted with R⁸-J-I and sodium hydride in DMF or with PS-BEMP (polystyrene-2-*tert*-butylimino-2-diethylamino-1,3-dimethyl-perhydro-1,3,2-diazaphosphorine) and R⁸-J-X (where X is I, Br, or Cl) in DMF to provide **24. 32** (or **30)** can also be reacted under Mitsunobu conditions (e.g., with DEAD (diethyl azodicarboxylate)) and PPh₃ (triphenylphoshine) in a solvent such as tetrahydrofuran (THF) with R⁸-OH to provide **24,** or **34,** respectively.

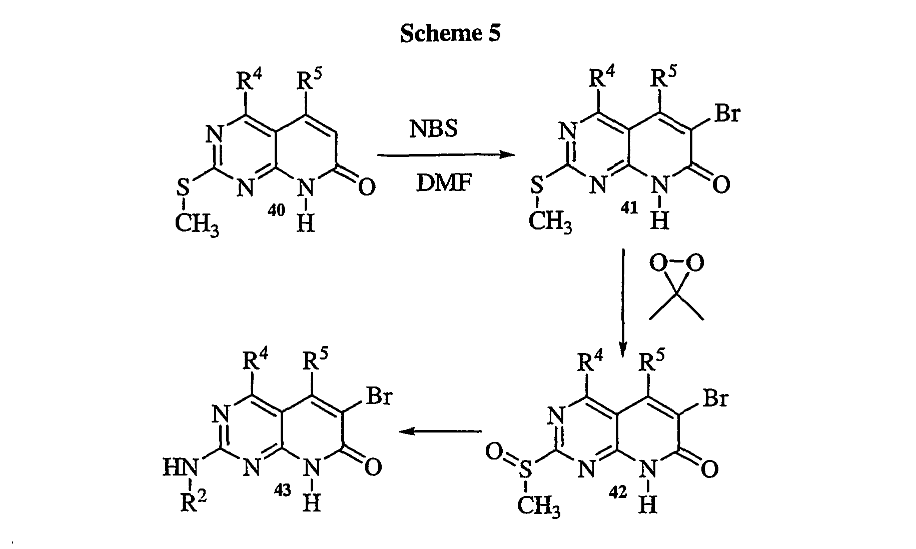

In Scheme **5, 40** is brominated with N-bromosuccinimide (NBS) in DMF to yield **41.** Alternatively, **40** can be chlorinated with N-chlorosuccinimide (NCS) to yield the chloro-analog of **41. 41** is then treated with dimethyldioxirane as in Scheme 2 to provide **42,** which is reacted in turn with **23** as in Scheme 2 to generate **43. 43** can then be reacted as in Scheme 4 with R⁸-J-I or R⁸-J-X to provide **44** (see Scheme 6).

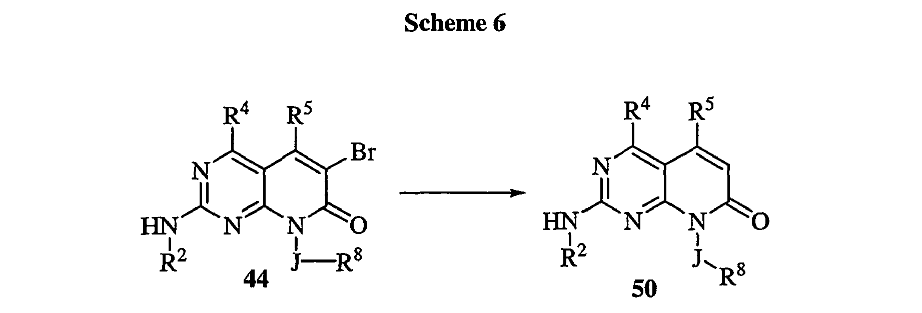

In Scheme 6, the bromo group of **44** can be reduced in a solvent such as a mixture of THF:methanol (1:1), with triethylamine and 10% Pd(OH)₂/C and under hydrogen gas (e.g., 50 p.s.i.) to provide **50.**

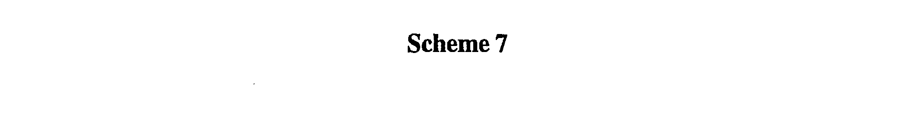

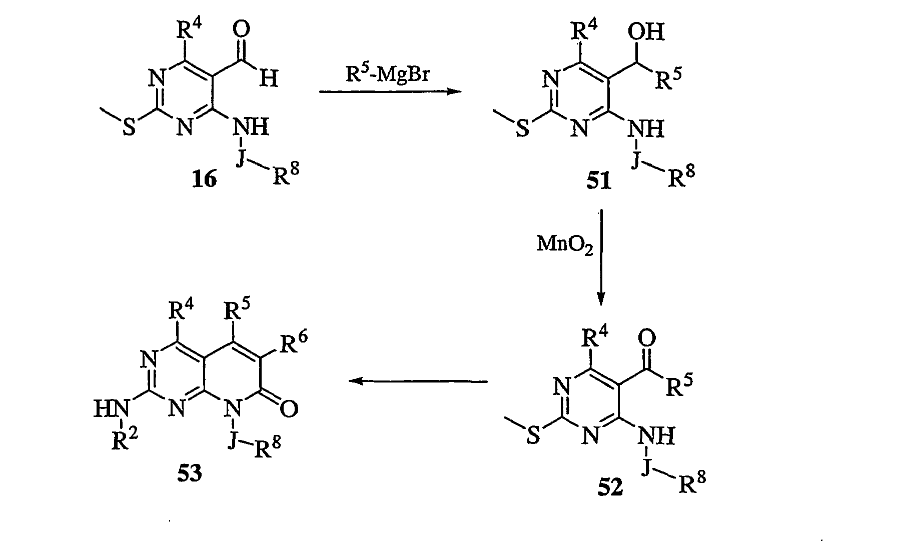

The aldehyde **16** can be reacted with an organometallic compound such as methyl magnesium bromide to give the alkylated alcohol **51.** This alcohol function can then be oxidized to the ketone **52** with an oxidizing reagent such as manganese dioxide. **52** can then be further converted to compounds of formula **53** according to the same methodology as scheme 2.

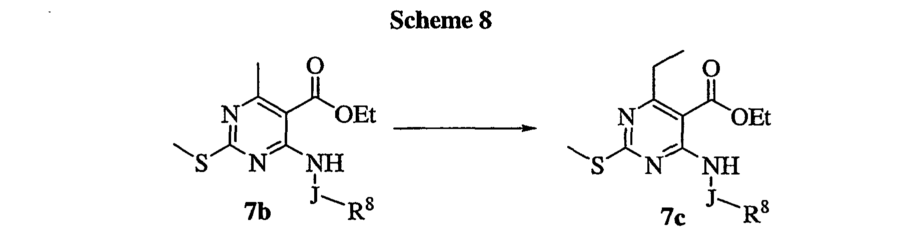

When a compound of formula **7** contains an R⁴ group with C-H bonds such as methyl, **7b,** the C-H bond is acidic and can be abstracted with a strong alkyl lithium reagent such as n-butyl lithium in THF, at cold temperatures (e.g., - 78°C) and alkylated with an alkyl iodide such as methyl iodide to give **7c. 7c** can then be used as in Scheme 2.

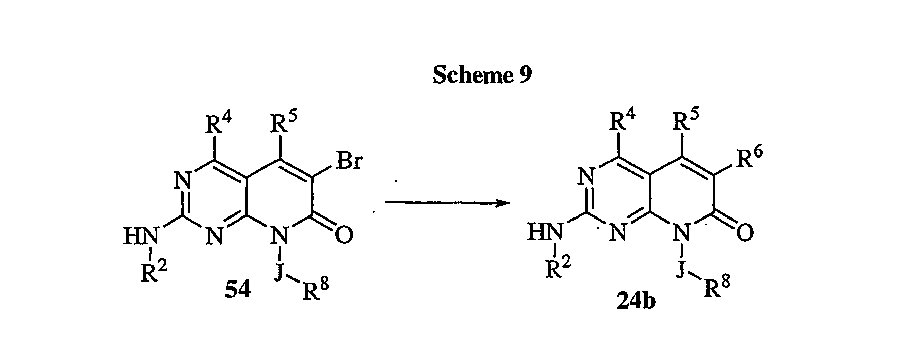

In Scheme **9, 54** can be coupled to a boronic acid compound R⁶-B(OH)₂ (e.g., phenylboronic acid), or an organic tin reagent such as trimethyl-tin R⁶, where R⁶ is a phenyl group, to generate **24b** (see generally, Miyaura and Suzuki, (1995) Chem. Rev. 95: 2457-2483). These reactions can be carried out using a phosphine-based palladium catalyst such as tetrakis(triphenylphosphine)palladium (Pd(Ph₃)₄) in a suitable solvent (e.g., aqueous sodium bicarbonate, methanol, and toluene (1:1:1)). Boronic acids are commercially available from vendors such as Sigma-Aldrich Corp., St. Louis, MO, USA. Alternatively, **20,** where R⁶ is Br, can be coupled to a boronic acid derivative followed by oxidation of the methyl sulfanyl group to a methyl sulfinyl group as in Scheme 2, followed displacement of the methyl sulfinyl group to provide **24b.**

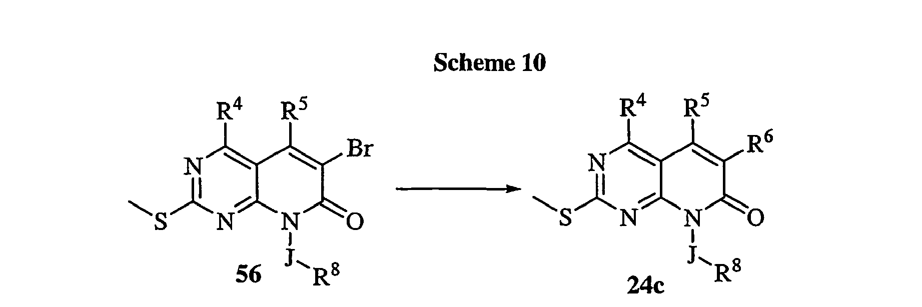

In Scheme 10, the C-6 position of **56** (e.g., 4-[6-bromo-8-(4-methoxybenzyl)-4-methyl-7-oxo-7,8-dihydro-pyrido[2,3-d]pyrimidin-2-ylamino]-1-methyl-1H-pyrrole-2-carboxylic acid methyl ester) can be alkylated with an organic tin reagent such as tetramethyl-tin or tetraethyl-tin, in the presence of copper iodide and PdCl₂(PPh₃)₂ in a solvent such as DMF to generate **24c** (e.g., 4-[8-(4-methoxy-benzyl)-4,6-dimethyl-7-oxo-7,8-dihydro-pyrido[2,3-*d*]pyrimidin-2-ylamino]-1-methyl-1*H*-pyrrole-2-carboxylic acid methyl ester).

### III. EVALUATION OF COMPOUNDS

Compounds of the present invention (e.g., compounds of Formulas I-III and pharmaceutically acceptable salts thereof) can be assayed for their ability to inhibit a PI3K. Examples of these assays are set out below and include in vitro and in vivo assays of PI3K activity.

In certain embodiments of the present invention are compounds that selectively inhibit one or more PI3Ks as compared to one or more enzymes including, but not limited to, a cyclic nucleotide dependent protein kinase, PDGF, a tyrosine kinase, a MAP kinase, a MAP kinase kinase, a MEKK, a cyclin-dependent protein kinase (e.g., CDK2/cyclinA). In other embodiments of the invention are compounds that selectively inhibit one PI3K as compared to another PI3K. For example, in certain embodiments, compounds of the present invention display the ability to selectively inhibit PI3Kγ as compared to PI3Kα or PI3Kβ. A compound selectively inhibits a first enzyme as compared to a second enzyme, when the IC₅₀ of the compound towards the first enzyme is less than the IC₅₀ of the compound towards the second compound. The IC₅₀ can be measured, for example, in an in vitro PI3K assay.

In presently preferred embodiments, compounds of the present invention can be assessed for their ability to inhibit PI3K activity in an in vitro or an in vivo assay (see below).

PI3K assays are carried out in the presence or absence of a PI3K inhibitory compound, and the amount of enzyme activity is compared for a determination of inhibitory activity of the PI3K inhibitory compound.

Samples that do not contain a PI3K inhibitory compound are assigned a relative PI3K activity value of 100. Inhibition of PI3K activity is achieved when the PI3K activity in the presence of a PI3K inhibitory compound is less than the control sample (i.e., no inhibitory compound). The IC₅₀ of a compound is the concentration of compound that exhibits 50% of the control sample activity. In certain embodiments, compounds of the present invention have an IC₅₀ of less than about 100 µM. In other embodiments, compounds of the present invention have an IC₅₀ of about 1 µM or less. In still other embodiments, compounds of the present invention have an IC₅₀ of about 200 nM or less.

PI3Kγ assays have been described in the art (see e.g., Leopoldt et al. J. Biol. Chem., 1998; 273: 7024-7029). Typically, a sample containing a complex of p101 and p110γ protein are combined with Gβ and Gγ proteins (e.g., G protein β₁/γ₂ subunits). Radiolabeled ATP (e.g., γ-³²P-ATP) is then added to this mixture. The lipid substrates are formed by creating PIP₂ containing lipid micelles. The reactions are then started by adding the lipid and enzyme mixtures and are stopped with the addition of H₃PO₄. The lipid products are then transferred to a glass fiber filter plate, and washed with H₃PO₄ several times. The presence of radioactive lipid product (PIP₃) can be measured using radiometric methods that are well-known in the art.

The activity of growth factor regulated PI3Ks can also be measured using a lipid kinase assay. For example, PI3Kα can be assayed using samples that contain a regulatory and a catalytic subunit. An activating peptide (e.g., pY peptide, SynPep Corp.) is added to the sample with radiolabeled ATP. PIP₂ containing lipid micelles are then added to the sample to start the reaction. The reactions are worked up and analyzed as described for the PI3Kγ assay just described. Assays can also be carried out using cellular extracts (Susa et al. J. Biol. Chem., 1992;267:22951-22956).

Compounds of the present invention (e.g., compounds of Formulas I-III and pharmaceutically acceptable salts thereof) can be assayed for their ability to decrease quantitative or qualitative markers of processes such as inflammation. Examples of animal models of arthritis are described below that may be used to assay the ability of a compound of the present invention to treat rheumatoid arthritis.

### Streptococcal cell wall (SCW) induced arthritis assay

Arthritis is induced as described by Schwab, et al., (1991) Infection and Immunity 59:4436-4442 with minor modifications. Rats typically about receive 6 µg sonicated SCW (in 10 µl Dulbecco's PBS (DPBS)) by an intraarticular injection into the right tibiotalar joint on day 0. On day 21, a delayed-type hypersensitivity reaction by systemic SCW can be initiated with about 100 µg of SCW (250 µl) administered i.v. For oral compound studies, compounds can be suspended in an appropriate vehicle (e.g., 0.5% hydroxypropylmethylcellulose/0.2% Tween 80), sonicated, and administered twice daily (10 ml/kg volume) beginning 1 hour prior to initiation of the delayed-type hypersensitivity reaction by i.v. injection of SCW. Compounds are typically administered in amounts between 10 and 500-mg/kg body weight/day, such as 20, 30, 60, 100, 200, and 300 mg/kg/day. Edema measurements are obtained by determining the baseline volumes of the sensitized hindpaw before reactivation on day 21, and comparing them with volumes at subsequent time points such as day 22, 23, 24, and 25. Mercury displacement plethysmography is one method that can be used to assay the paw volume of an animal.

To measure pain, rats are placed in a device that measures the amount of pressure placed on each hind paw. The average difference between the arthritic and normal paws is one method used to measure pain. In addition, inflammatory cytokine levels can be measured in material that has been lavaged from the arthritic joints of an animal and compared to the levels of a non-arthritic joint or typical non-arthritic joint levels.

### Collagen-induced arthritis assay

Type II collagen-induced arthritis (CIA) in mice is an experimental model of rheumatoid arthritis (see e.g., Stuart et al. (1984) Annual Rev. Immunol. 2: 199-218; Wooley (1988) Meth. Enzymol. 162: 361-373). The disease is typically induced by immunization of DBA/1 mice with 100 µg of type II collagen ("CII") (e.g., bovine or chick type II collagen), delivered intradermally at the base of the tail in Freund's complete adjuvant.

A progressive and inflammatory arthritis develops in the majority of mice immunized, characterized by paw width increases of up to 100%. A test compound can be administered to mice in a range of amounts, such as 20, 60, 100, and 200 mg/kg body weight/day. The duration of the test can be several weeks to a few months, such as 40, 60, or 80 days. A clinical scoring index can be used to assess disease progression from erythema and edema (stage 1), joint distortion (stage 2), to joint ankylosis (stage 3). The disease can affect one or all paws in an animal, resulting in a total possible score of 12 for each mouse. Histopathology of an arthritic joint generally reveals synovitis, pannus formation, and cartilage and bone erosions. All mouse strains that are susceptible to CIA are high antibody responders to type II collagen, and there is a marked cellular response to CII.

Dosing of a test compound is usually performed either prophylactically (for 10 weeks) or therapeutically (for 10 days when disease is first observed). Mice are typically examined daily for the development of arthritis and a clinical score is often assigned. Serum can also retrieved from each animal at various time points for measurement of antibodies or cytokines (as required). At the end of the study the mice can be euthanized and a quantitative histopathology score assigned.

### Monoclonal Antibody-Induced Arthritis assay

Another experimental model of rheumatoid arthritis involves injecting antibodies to type II collagen epitopes into a mouse to induce arthritis in a few days (see e.g., Burkhardt et al. (2002) Arthritis & Rheumatism 46: 2339-2348; Terato et la. (1992) J. Immunol. 148: 2103-2108). Cocktails of four antibodies are commercially available for use in this model (Arthrogen-CIA® Monoclonal Antibody Cocktail, CHEMICON International, Inc., Temecula, CA). This model does not require DBA/1 strain mice. A test compound can be administered to mice one or more times in a range of amounts, such as 20, 60, 100, and 200 mg/kg body weight/day at any time during the study. Ankle and paw swelling are typically measured quantitatively or qualitatively as endpoints as well as histology. In addition, serum can be retrieved from each animal at various time points for measurement of antibodies or cytokines.

### IV. PHARMACEUTICALLY ACCEPTABLE SALTS AND SOLVATES

The compounds to be used in the present invention can exist in unsolvated forms as well as solvated forms, including hydrated forms. In general, the solvated forms, including hydrated forms, are equivalent to unsolvated forms and are intended to be encompassed within the scope of the present invention.

The compounds of the present invention (e.g., compounds of Formulas I-III) are capable of further forming both pharmaceutically acceptable salts, including but not limited to acid addition and/or base salts. Pharmaceutically acceptable salts of the compounds of formula (I) include the acid addition and base salts (including disalts) thereof. Examples of suitable salts can be found for example in Stahl and Wermuth, Handbook of Phannaceutical Salts: Properties, Selection, and Use, Wiley-VCH, Weinheim, Germany (2002); and Berge et al., "Pharmaceutical Salts," J. of Pharmaceutical Science, 1977;66:1-19.

Pharmaceutically acceptable acid addition salts of the compounds of Formulas I-III include non-toxic salts derived from inorganic acids such as hydrochloric, nitric, phosphoric, sulfuric, hydrobromic, hydriodic, phosphorus, and the like, as well as the salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include the acetate, aspartate, benzoate, besylate (benzenesulfonate), bicarbonate/carbonate, bisulfate, caprylate, camsylate (camphor sulfonate), chlorobenzoate, citrate, edisylate (1,2-ethane disulfonate), dihydrogenphosphate, dinitrobenzoate, esylate (ethane sulfonate), fumarate, gluceptate, gluconate, glucuronate, hibenzate, hydrochloride/chloride, hydrobromide/bromide, hydroiodide/iodide, isobutyrate, monohydrogen phosphate, isethionate, D-lactate, L-lactate, malate, maleate, malonate, mandelate, mesylate (methanesulfonate), metaphosphate, methylbenzoate, methylsulfate, 2-napsylate (2-naphthalene sulfonate), nicotinate, nitrate, orotate, oxalate, palmoate, phenylacetate, phosphate, phthalate, propionate, pyrophosphate, pyrosulfate, saccharate, sebacate, stearate, suberate, succinate sulfate, sulfite, D-tartrate, L-tartrate, tosylate (toluene sulfonate), and xinafoate salts, and the like of compounds of Formulas I-III. Also contemplated are the salts of amino acids such as arginate, gluconate, galacturonate, and the like.

Acid addition salts of the basic compounds may be prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention.

Pharmaceutically acceptable base addition salts may be formed with metals or amines, such as alkali and alkaline earth metal hydroxides, or of organic amines. Examples of metals used as cations are aluminum, calcium, magnesium, potassium, sodium, and the like. Examples of suitable amines include arginine, choline, chloroprocaine, N,N'-dibenzylethylenediamine, diethylamine, diethanolamine, diolamine, ethylenediamine (ethane-1,2-diamine), glycine, lysine, meglumine, N-methylglucamine, olamine, procaine (benzathine), and tromethamine.

The base addition salts of acidic compounds may be prepared by contacting the free acid form with a sufficient amount of the desired base to produce the salt in the conventional manner. The free acid form may be regenerated by contacting the salt form with an acid and isolating the free acid in a conventional manner. The free acid forms differ from their respective salt forms somewhat in certain physical properties such as solubility in polar solvents, but otherwise the salts are equivalent to their respective free acid for purposes of the present invention.

### V. PHARMACEUTICAL COMPOSITIONS AND METHODS OF ADMINISTRATION

This invention also provides for pharmaceutical compositions comprising a therapeutically effective amount of a compound of Formulas I-III, or a pharmaceutically acceptable salt thereof together with a pharmaceutically acceptable carrier, diluent, or excipient therefor. The phrase "pharmaceutical composition" refers to a composition suitable for administration in medical or veterinary use. The phrase "therapeutically effective amount" means an amount of a compound, or a pharmaceutically acceptable salt thereof, sufficient to inhibit, halt, or allow an improvement in the disease being treated when administered alone or in conjunction with another pharmaceutical agent or treatment in a particular subject or subject population. For example in a human or other mammal, a therapeutically effective amount can be determined experimentally in a laboratory or clinical setting, for the particular disease and subject being treated.

It should be appreciated that determination of proper dosage forms, dosage amounts, and routes of administration is within the level of ordinary skill in the pharmaceutical and medical arts, and is described below.

A compound of the present invention can be formulated as a pharmaceutical composition in the form of a syrup, an elixir, a suspension, a powder, a granule, a tablet, a capsule, a lozenge, a troche, an aqueous solution, a cream, an ointment, a lotion, a gel, an emulsion, etc. Preferably, a compound of the present invention will cause a decrease in symptoms or a disease indicia associated with a PI3K-mediated disease as measured quantitatively or qualitatively.

For preparing pharmaceutical compositions from the compounds of the present invention, pharmaceutically acceptable carriers can be either solid or liquid. Solid form preparations include powders, tablets, pills, capsules, cachets, suppositories, and dispersible granules. A solid carrier can be one or more substances which may also act as diluents, flavoring agents, binders, preservatives, tablet disintegrating agents, or an encapsulating material.

In powders, the carrier is a finely divided solid which is in a mixture with the finely divided active component. In tablets, the active component is mixed with the carrier having the necessary binding properties in suitable proportions and compacted in the shape and size desired.

The powders and tablets contain from 1% to 95% (w/w) of the active compound. In certain embodiments, the active compound ranges from 5% to 70% (w/w). Suitable carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

For preparing suppositories, a low melting wax, such as a mixture of fatty acid glycerides or cocoa butter, is first melted and the active component is dispersed homogeneously therein, as by stirring. The molten homogeneous mixture is then poured into convenient sized molds, allowed to cool, and thereby to solidify.

Liquid form preparations include solutions, suspensions, and emulsions, for example, water or water/propylene glycol solutions. For parenteral injection, liquid preparations can be formulated in solution in aqueous polyethylene glycol solution.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations which are intended to be converted, shortly before use, to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, solubilizing agents, and the like.

The pharmaceutical preparation is preferably in unit dosage form. In such form the preparation is subdivided into unit doses containing appropriate quantities of the active component. The unit dosage form can be a packaged preparation, the package containing discrete quantities of preparation, such as packeted tablets, capsules, and powders in vials or ampules. Also, the unit dosage form can be a capsule, tablet, cachet, or lozenge itself, or it can be the appropriate number of any of these in packaged form.

The quantity of active component in a unit dose preparation may be varied or adjusted from 0.1 mg to 1000 mg, preferably 1.0 mg to 100 mg, or from 1% to 95% (w/w) of a unit dose, according to the particular application and the potency of the active component. The composition can, if desired, also contain other compatible therapeutic agents.

Pharmaceutically acceptable carriers are determined in part by the particular composition being administered, as well as by the particular method used to administer the composition. Accordingly, there are a wide variety of suitable formulations of pharmaceutical compositions of the present invention (see, e.g., Remington: The Science and Practice of Pharmacy, 20th ed., Gennaro et al. Eds., Lippincott Williams and Wilkins, 2000).

A compound of the present invention, alone or in combination with other suitable components, can be made into aerosol formulations (i.e., they can be "nebulized") to be administered via inhalation. Aerosol formulations can be placed into pressurized acceptable propellants, such as dichlorodifluoromethane, propane nitrogen, and the like.

Formulations suitable for parenteral administration, such as, for example, by intravenous, intramuscular, intradermal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and nonaqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. The formulations of compounds can be presented in unit-dose or multi-dose sealed containers, such as ampules and vials. Injection solutions and suspensions can be prepared from sterile powders, granules, and tablets of the kind previously described.

The dose administered to a subject, in the context of the present invention should be sufficient to affect a beneficial therapeutic response in the subject over time. The term "subject" refers to a member of the class Mammalia. Examples of mammals include, without limitation, humans, primates, chimpanzees, rodents, mice, rats, rabbits, horses, livestock, dogs, cats, sheep, and cows.

The dose will be determined by the efficacy of the particular compound employed and the condition of the subject, as well as the body weight or surface area of the subject to be treated. The size of the dose also will be determined by the existence, nature, and extent of any adverse side-effects that accompany the administration of a particular compound in a particular subject. In determining the effective amount of the compound to be administered in the treatment or prophylaxis of the disease being treated, the physician can evaluate factors such as the circulating plasma levels of the compound; compound toxicities, and/or the progression of the disease, etc. In general, the dose equivalent of a compound is from about 1 µg/kg to 100 mg/kg for a typical subject. Many different administration methods are known to those of skill in the art.

For administration, compounds of the present invention can be administered at a rate determined by factors that can include, but are not limited to, the pharmacokinetic profile of the compound, contraindicated drugs, and the side-effects of the compound at various concentrations, as applied to the mass and overall health of the subject. Administration can be accomplished via single or divided doses.

Examples of a typical tablet, parenteral, and patch formulation include the following:

### TABLET FORMULATION EXAMPLE 1

| Tablet Formulation | |
|---|---|
| Ingredient | Amount |
| A Compound of Formulas I | 50 mg |
| Lactose | 80 mg |
| Cornstarch (for mix) | 10 mg |
| Cornstarch (for paste) | 8 mg |
| Magnesium Stearate (1%) | 2 mg |
| | 150 mg |

The compounds of the present invention (e.g., a compound of Formulas I, or a pharmaceutically acceptable salt thereof) can be mixed with the lactose and cornstarch (for mix) and blended to uniformity to a powder. The cornstarch (for paste) is suspended in 6 mL of water and heated with stirring to form a paste. The paste is added to the mixed powder, and the mixture is granulated. The wet granules are passed through a No. 8 hard screen and dried at 50°C. The mixture is lubricated with 1% magnesium stearate and compressed into a tablet. The tablets are administered to a patient at the rate of 1 to 4 each day for treatment of a PI3K-mediated disease.

### PARENTERAL SOLUTION FORMULATION EXAMPLE 1

In a solution of 700 mL of propylene glycol and 200 mL of water for injection can be added 20.0 g of a compound of the present invention. The mixture is stirred, and the pH is adjusted to 5.5 with hydrochloric acid. The volume is adjusted to 1000 mL with water for injection. The solution is sterilized, filled into 5.0 mL ampules, each containing 2.0 mL (40 mg of invention compound), and sealed under nitrogen. The solution is administered by injection to a subject suffering from a PI3K-mediated disease and in need of treatment.

### PATCH FORMULATION EXAMPLE 1

Ten milligrams of a compound of the present invention can be mixed with 1 mL of propylene glycol and 2 mg of acrylic-based polymer adhesive containing a resinous cross-linking agent. The mixture is applied to an impermeable backing (30 cm²) and applied to the upper back of a patient for sustained release treatment of a PI3K-mediated disease.

### VI. METHODS FOR TREATING PI3K-MEDIATED DISEASES

The compounds of the present invention and pharmaceutical compositions comprising a compound of the present invention can be administered to a subject suffering from a PI3K-mediated disease. PI3K-mediated diseases can be treated prophylactically, acutely, and chronically using compounds of the present invention, depending on the nature of the disease. Typically, the host or subject in each of these methods is human, although other mammals can also benefit from the administration of a compound of the present invention.

In therapeutic applications, the compounds of the present invention can be prepared and administered in a wide variety of oral and parenteral dosage forms. The term "administering" refers to the method of contacting a compound with a subject. Thus, the compounds of the present invention can be administered by injection, that is, intravenously, intramuscularly, intracutaneously, subcutaneously, intraduodenally, parentally, or intraperitoneally. Also, the compounds described herein can be administered by inhalation, for example, intranasally. Additionally, the compounds of the present invention can be administered transdermally, topically, via implantation, transdermally, topically, and via implantation. In certain embodiments, the compounds of the present invention are delivered orally. The compounds can also be delivered rectally, bucally, intravaginally, ocularly, andially, or by insufflation.

The compounds utilized in the pharmaceutical method of the invention can be administered at the initial dosage of about 0.001 mg/kg to about 100 mg/kg daily. In certain embodiments, the daily dose range is from about 0.1 mg/kg to about 10 mg/kg. The dosages, however, may be varied depending upon the requirements of the subject, the severity of the disease being treated, and the compound being employed. Determination of the proper dosage for a particular situation is within the skill of the practitioner. Generally, treatment is initiated with smaller dosages, which are less than the optimum dose of the compound. Thereafter, the dosage is increased by small increments until the optimum effect under circumstances is reached. For convenience, the total daily dosage may be divided and administered in portions during the day, if desired. The term "treatment" includes the acute, chronic, or prophylactic diminishment or alleviation of at least one symptom or characteristic associated with or caused by the disease being treated. For example, treatment can include diminishment of several symptoms of a disease, inhibition of the pathological progression of a disease, or complete eradication of a disease. The compounds of the present invention can be co-administered to a subject. The term "co-administered" means the administration of two or more different pharmaceutical agents or treatments (e.g., radiation treatment) that are administered to a subject by combination in the same pharmaceutical composition or separate pharmaceutical compositions. Thus co-administration involves administration at the same time of a single pharmaceutical composition comprising two or more pharmaceutical agents or administration of two or more different compositions to the same subject at the same or different times. For example, a subject that is administered a first dosage that comprises a compound of the present invention at 8 a.m. and then is administered a second therapeutic agent at 1-12 hours later, e.g., 6 p.m., of that same day has been co-administered with a compound of the present invention and the second therapeutic agent. Alternatively, for example, a subject could be administered with a single dosage comprising a compound of the presents invention and a second therapeutic agent at 8 a.m. has been co-administered with a compound of the present invention and the second therapeutic agent.

Thus, compounds of the invention can also be co-administered with compounds that are useful for the treatment of cancer (e.g., cytotoxic drugs such as TAXOL®, taxotere, GLEEVEC® (Imatinib Mesylate), adriamycin, daunomycin, cisplatin, etoposide, a vinca alkaloid, vinblastine, vincristine, methotrexate, or adriamycin, daunomycin, cis-platinum, etoposide, and alkaloids, such as vincristine, farnesyl transferase inhibitors, endostatin and angiostatin, VEGF inhibitors, and antimetabolites such as methotrexate. The compounds of the present invention may also be used in combination with a taxane derivative, a platinum coordination complex, a nucleoside analog, an anthracycline, a topoisomerase inhibitor, or an aromatase inhibitor). Radiation treatments can also be co-administered with a compound of the present invention for the treatment of cancers.

The compounds of the invention can also be co-administered with compounds that are useful for the treatment of a thrombolytic disease, heart disease, stroke, etc., (e.g., aspirin, streptokinase, tissue plasminogen activator, urokinase, anticoagulants, antiplatelet drugs (e.g., PLAVIX®; clopidogrel bisulfate), a statin (e.g., LIPITOR® (Atorvastatin calcium), ZOCOR® (Simvastatin), CRESTOR® (Rosuvastatin), etc.), a Beta blocker (e.g, Atenolol), NORVASC® (amlodipine besylate), and an ACE inhibitor (e.g., Accupril® (Quinapril Hydrochloride), Lisinopril, etc.).

The compounds of the invention can also be co-administered for the treatment of hypertension with compounds such as ACE inhibitors, lipid lowering agents such as statins, LIPITOR® (Atorvastatin calcium), calcium channel blockers such as NORVASC® (amlodipine besylate). The compounds of the present invention may also be used in combination with fibrates, beta-blockers, NEPI inhibitors, Angiotensin-2 receptor antagonists and platelet aggregation inhibitors.

For the treatment of inflammatory diseases, including rheumatoid arthritis, the compounds of the invention may be co-administered with agents such as TNF-α inhibitors such as anti-TNFα monoclonal antibodies (such as REMICADE®, CDP-870 and HUMIRA^{™} (adalimumab) and TNF receptor-immunoglobulin fusion molecules (such as ENBREL®), IL-1 inhibitors, receptor antagonists or soluble IL-IRα (e.g. KINERET^{™} or ICE inhibitors), nonsteroidal anti-inflammatory agents (NSAIDS), piroxicam, diclofenac, naproxen, flurbiprofen, fenoprofen, ketoprofen ibuprofen, fenamates, mefenamic acid, indomethacin, sulindac, apazone, pyrazolones, phenylbutazone, aspirin,COX-2 inhibitors (such as CELEBREX® (celecoxib), BEXTRA® (valdecoxib) and etoricoxib, metalloprotease inhibitors (preferably NEMP-13 selective inhibitors), NEURONTIN®, pregabalin, low dose methotrexate, leflunomide, hydroxychloroquine, d-penicillamine, auranofin or parenteral or oral gold.

The compounds of the invention may be co-administered with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flurbiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, and etoricoxib, analgesics and intraarticular therapies such as corticosteroids and hyaluronic acids such as hyalgan and synvisc.

The compounds of the invention may also be co-administered with antiviral agents such as Viracept, AZT, acyclovir and famcyclovir, and antisepsis compounds such as Valant.

The compounds of the present invention may further be co-administered with CNS agents such as antidepressants (such as sertraline), anti-Parkinsonian drugs (such as deprenyl, L-Dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), NEURONTIN®, pregabalin, and anti-Alzheimer's drugs such as ARICEPT®, tacrine, propentofylline or metrifonate.

The compounds of the present invention may additionally be co-administered with osteoporosis agents such as EVISTA® (raloxifene hydrochloride) droloxifene, lasofoxifene or FOSAMAX® and immunosuppressant agents such as FK-506 and rapamycin.

### EXAMPLES

Intermediate 1: **1-(1-Dimethylamino-ethylidene)-2-methyl-isothiourea.** To a solution of dichloromethane (10 ml) and thiourea (0.761 g, 10 mmol) was added (1,1-dimethoxy-ethyl)-dimethyl-amine (1.9 ml, 13 mmol). The reaction heated to reflux for 4 hours, then cooled and the dichloromethane was removed under reduced pressure to give an orange solid. The solid was titurated with hot ethyl ether and dried. This intermediate was stirred in a 1:1 mixture of THF(tetrahydrofuran)/MeI (10 ml) for 18 hours at room temperature. The slurry was filtered and rinsed with ethyl ether to give the title compound as a colorless solid, 2.54 g. ¹H NMR (DMSO): 9.15 (brs,1H), 8.80 (s,1H), 3.18 (s, 3H),3.08 (s,3H)2.35 (s,3H), 2.23 (s,3H).

Intermediate 2: **4-Hydroxy-6-methyl-2-methylsulfanyl-pyrimidine-5-carboxylic acid ethyl ester.** To slurry of Intermediate 1 (2.86 g, 10 mmol) in dichloromethane (50 ml) at room temperature was added chlorocarbonyl-acetic acid ethyl ester (1.54 ml, 12 mmol). After the reaction stirred for four hours giving an orange suspension, it was cooled to 0°C and added triethylamine (3.34 ml, 24 mmol). The reaction mixture warmed to room temperature and stirred for 24 hours. The mixture was then poured into dichloromethane, and then washed successively with 10% sulfuric acid, water, and brine. The organic phase was then dried over magnesium sulfate and the dichloromethane was removed under reduced pressure to give a yellow solid, 2.12 g. The crude product was crystallized from dichloromethane/ hexanes to give the title compound as a light yellow solid, 1.05 g. ¹H NMR (CDCl₃): (4.41 (q,2H), 2.59 (s,3M, 2.57 (s,3H), 1.41 (t,3H).

Intermediate 3: **4-Chloro-6-methyl-2-methylsulfanyl-pyrimidine-5-carboxylic acid ethyl ester.** To a solution of dichloromethane (250 ml) and Intermediate 2 (15.0 g, 65.7 mmol) was added oxalyl chloride (11.47 ml, 131.4 mmol) and a catalytic amount of DMF (N,N-dimethylformamide). A precipitate formed immediately and some gas was evolved. The mixture was stripped two times with dichloromethane to afford the title compound. ¹H NMR (CDCl₃) 4.42 (q,2H), 2.57 (s,3H), 2.48 (s,3H), 1.39 (t,3H). MS (M+1): 247.

Intermediate 4: **4-Cycloheptylamino-6-methyl-2-methyl-sulfanyl-pyrimidine-5-carboxylic acid ethyl ester.** To a solution of dichloromethane (15 ml) and Intermediate 3 (1.02 g, 4.1 mmol) was added triethylamine (1.73 ml, 12.3 mmol) and cycloheptylamine (0.58 ml, 4.51 mmol). The reaction mixture stirred overnight at room temperature. The reaction mixture was poured into water and dichloromethane and added 1.0 N hydrochloric acid (8.0 ml), washed with water, brine, dried over magnesium sulfate, removed dichloromethane under reduced pressure to give the title compound as a brown oil, 1.13 g. ¹H NMR(CDCl₃): 4.32 (q,2H), 4.25 (brs,1H), 2.50 (m,6H), 1.98 (m,1H), 1.60 (m,10H), 1.40 (t,3H). MS(M+1): 324.

Intermediate 5: **4-Cycloheptylamino-6-methyl-2-methyl-sulfanyl-pyrimidin-5-yl)-methanol.** To a solution of THF (40 ml) and Intermediate 4 (1.0 g, 3.1 mmol) was added lithium aluminum hydride (0.117 g, 3.1 mmol). The reaction mixture stirred at room temperature for one hour and was monitored by TLC (thin-layer chromatography) (10% ethyl acetate/dichloromethane). The reaction was quenched with 2% sodium hydroxide, filtered and the THF was removed under reduced pressure to give the title compound as an orange solid, 0.854 g. ¹H NMR (CDCl₃): 5.87 (d,1H), 4.60 (s,2H), 4.18 (brs,1H), 2.50 (s,3H), 2.21 (s,3H), 1.97 (m,1H), 1.70-1.40 (m,10H). MS (M+1) (282).

Intermediate 6: **4-Cycloheptylamino-6-methyl-2-methyl sulfanyl-pyrimidin-5-carbaldehyde.** To a solution of chloroform (30 ml) and Intermediate 5 (0.854 g, 3.0 mmol) was added manganese dioxide (2.11 g, 24 mmol). The reaction mixture was heated to reflux for 3 hours. The manganese dioxide was filtered away and the filtrate was concentrated to give the title compound as a colorless solid, 0.721 g. ¹H NMR (CDCl₃): 10.18 (s,1H), 9.13 (s,1H), 4.30 (m,1H), 2.58 (s,3H), 2.50 (s,3H), 1.97 (m,1H), 1.70-1.50 (m,10H). MS(M+1): 280. Intermediate 7: **3-(4-Cycloheptylamino-6-methyl-2-methyl-sulfanyl-pyrimidin-5-yl)-acrylic acid ethyl ester.** To a solution of THF (10 ml) and ethoxyphosphinoyl-acetic acid ethyl ester (0.715 ml, 3.6 mmol) was added sodium hydride (0.083 g, 3.45 mmol). The reaction mixture stirred at room temperature for 20 minutes, then added Intermediate 6 (0.838 g, 3.0 mmol) in THF (15 ml) to the reaction mixture. The mixture was heated to reflux for several hours and was monitored by TLC (9:1 hexanes/ethyl ether). The TLC showed mostly starting material, however a mass spectrometric analysis revealed mostly product. The reaction was cooled to room temperature and poured into water. Ethyl acetate was added to the mixture, which was then successively washed one time each with water, bicarbonate, and then brine. The solution was then dried over magnesium sulfate, and the ethyl acetate was removed under reduced pressure to give the title compound as a colorless oil, 1.07 g. ¹H NMR (CDCl₃): 7.61 (d,1H), 6.18 (d,1H), 5.10 (s,1H), 4.30 (q,2H), 4.20 (m,1H), 4.4.55 (s,3H), 4.18 (s,3H), 2.00 (m,1H), 1.70-1,47 (m,10H), 1.35 (t,3H). MS(M+1): 350.

Intermediate 8: **8-Cycloheptyl-4-methyl-2-methylsulfanyl-8H-pyrido[2,3-d]pyrimidin-7-one.** To a solution of DMF (4 ml) and Intermediate 7 (0.860 g, 2.46 mmol) was added DBU (1,8-diazabicyclo[5.4.0]undec-7-ene) (0.410 ml, 2.70 mmol) and a catalytic amount of potassium *tert*-butoxide. The reaction mixture was heated to 150°C for 24 hours, then cooled to room temperature for 72 hours. The reaction mixture was poured into water, and then acidified to pH=4 with 1 equivalent of 10% sulfuric acid. The reaction was extracted with ethyl acetate, and then washed successively with water and brine. The mixture was then dried with magnesium sulfate and the ethyl acetate was removed under reduced pressure to give the title compound as an orange oil, 0.590 g. ¹H NMR (CDCl₃): 7.73 (d,1H), 6.58 (brd, 1H), 5.78,5.40 (brs,1H), 2.63 (s,3H), 2.53 (m,3H), 1.90-1.50 (M,11H). MS(M+1): 304.

Intermediate 9: **8-Cycloheptyl-4-methyl-2-methylsulfinyl-8H-pyrido[2,3-d]pyrimidin-7-one.** To a solution of Intermediate 8 (590 mg, 2.0 mmol) in chloroform (15 ml) was added Davis oxaziridine (533 mg, 1.1 equiv). The reaction was stirred at room temperature for 2 hours. Thin layer analysis at this point indicated that the reaction was nearly complete. The solvent was partially removed under reduced pressure and the reaction mixture was chromatographed on silica gel eluting with ethyl acetate then 10% methanol in ethyl acetate to give the title compound as a colorless foam, 400 mg. ¹H NMR: 7.81 (d,1H), 6.78 (brd,1H), 2.95 (s,3H), 2.81 (s,3H), 2.52 (m,1H), 1.90-1.50 (m,11H). MS(M+1): 320.

Example 1: **4-(8-Cycloheptyl-4-methyl-7-oxo-7,8-dihydro-pyrido[2,3-d]pyrimidin-2-ylamino)-1-methyl-1H-pyrrole-2-carboxylic acid methyl ester.** A mixture of Intermediate 9 (0.4 g, 1.25 mmol), 4-amino-1-methylpyrrole-2-carboxylic acid methyl ester.HCl (0.475 g, 2.5 mmol), triethylamine (0.348 ml, 2.5 mmol) and acetonitrile (4 ml) was heated to 120°C for 18 hours allowing the solvent to distill away. The residue was dissolved in ethyl acetate, extracted with 1N HCl, dried over MgSO_{4,} filtered and concentrated under reduced pressure. The residue was purified on a silica gel column using THF/ hexane as a mobile phase to afford the title compound. Yield: 0.25 g, 48%.

The title compounds of Examples 2-8 were synthesized in a manner analogous to Example 1.

Example 2: **4-(4,8-Dimethyl-7-oxo-7,8-dihydro-pyrido[2,3-d]pyrimidin-2-ylamino)-1-(1-phenyl-ethyl)-1H-pyrrole-2-carboxylic acid ethyl ester.**

Example 3: **4-(8-Cyclopropyl-4-methyl-7-oxo-7,8-dihydro-pyrido[2,3-d]pyrimidin-2-ylamino)-1-methyl-1H-pyrrole-2-carboxylic acid methyl ester.**

Example 4: **4-(8-Cyclobutyl-4-methyl-7-oxo-7,8-dihydro-pyrido[2,3-d]pyrimidin-2-ylamino)-1-methyl-1H-pyrrole-2-carboxlylic acid methyl ester.**

Example 5: **1-Methyl-4-[4-methyl-7-oxo-8-(tetrahydro-pyran-4-yl)-7,8-dihydro-pyrido[2,3-d]pyrimidin-2-ylamino]-1H-pyrrole-2-carboxylic acid methyl ester.**

Example 6: **4-(8-Cyclohexyl-4-methyl-7-oxo-7,8-dihydro-pyrido[2,3-d]pyrimidin-2-ylamino)-1-methyl-1H-pyrrole-2-carboxylic acid methyl ester.**

Example 7: **1-Benzyl-4-(8-cycloheptyl-4-methyl-7-oxo-7,8-dihydro-pyrido[2,3-d]pyrimidin-2-ylamino)-1H-pyrrole-2-carboxylic acid ethyl ester.**

Example 8: **4-[8-(2-Cyclopropyl-ethyl)-4-methyl-7-oxo-7,8-dihydro-pyrido[2,3-d]pyrimidin-2-ylaminol-1-methyl-1H-pyrrole-2-carboxylic acid methyl ester.**

The title compounds of Examples 9 through 13 were synthesized in a manner analogous to Example 1 by replacing ethoxyphosphinoyl-acetic acid ethyl ester with triethyl 2-fluoro-2-phosphonoacetate.

Example 9: **4-(8-Cyclopentyl-6-fluoro-4-methyl-7-oxo-7,8-dihydro-pyrido[2,3-d]pyrimidin-2-ylamino)-1-methyl-1H-pyrrole-2-carboxylic acid methyl ester.**

Example 10: **4-[6-Fluoro-4-methyl-7-oxo-8-(tetrahydro-pyran-4-ylmethyl)-7,8-dihydro-pyrido[2,3-d]pyrimidin-2-ylamino]-1-methyl-1H-pyrrole-2-carboxylic acid methyl ester.**

Example 11: **4-[6-Fluoro-8-(4-methoxy-cyclohexyl)-4-methyl-7-oxo-7,8-dihydro-pyrido[2,3-d]pyrimidin-2-ylamino]-1-methyl-1H-pyrrole-2-carboxylic acid methyl ester.**

Example 12: **4-(8-Cyclopentyl-6-fluoro-4-methyl-7-oxo-7,8-dihydro-pyrido[2,3-d]pyrimidin-2-ylamino)-1-pyridin-3-ylmethyl-1H-pyrrole-2-carboxylic acid ethyl ester.**

Example 13: **4-(8-Cyclopentyl-6-fluoro-4-methyl-7-oxo-7,8-dihydro-pyrido[2,3-d]pyrimidin-2-ylamino)-1-(1-phenyl-ethyl)-1H-pyrrole-2-carboxylic acid ethyl ester.** Intermediate 10. **4-Cycloheptylamino-6-ethyl-2-methylsulfanyl-pyrimidine-5-carboxylic acid ethyl ester.** A solution of diisopropyl amine (3.25 ml, 23.2 mmol) in THF (90 ml) was cooled to 0°C and treated dropwise with n-BuLi (15.2 ml, 1.6 M, 24 mmol). The solution was warmed to ambient temperature for 0.5 hours and then cooled to -78 °C. A solution of cycloheptylamino-6-methyl-2-methyl-sulfanyl-pyrimidine-5-carboxylic acid ethyl ester (3.0 g, 9.28 mmol) in THF (30 ml) was added dropwise to the mixture and the solution stirred for 2 hour at -78°C. Methyl iodide (0.186 ml, 3 mmol) was added to the reaction and mixture was allowed to warm to ambient temperature, diluted with ethyl acetate and extracted with 1N HCl. The solution was dried, concentrated under reduced pressure and the residue was purified on a silica gel column (Hexane/Ethyl Acetate) to afford 2.7 g of the title compound (86%). ¹H NMR (400 MHz, DMSO-D6): δ ppm 1.1 (t, *J*=7.4 Hz, 3 H) 1.3 (t, *J*=7.1 Hz, 3 H) 1.5 (m, 2 H) 1.5 (m, 8 H) 1.9 (m, 2 H) 2.5 (s,3 H) 2.8 (m, 2 H) 4.2 (m, 1 H) 4.3 (q, *J*=7.1 Hz, 2 H)

Intermediate 11: **8-Cycloheptyl-4-ethyl-2-methylsulfinyl-8H-pyrido[2,3-d]pyrimidin-7-one.** Intermediate 11 was synthesized from Intermediate 10 in a manner analogous to the synthesis of Intermediate 9. ¹H NMR (400 MHz, DMSO-D6): δ ppm 1.2 (t, *J*=7.6 Hz, 3 H) 1.5 (m, 3 H) 1.6-1.8 (m, 12 H) 2.9 (s, 3 H) 3.1 (q, *J*=7.6 Hz, 3 H) 6.8 (m, 1 H) 7.5 (d, *J*=7.8 Hz, 1 H) 8.2 (d, *J*=9.8 Hz, 1 H)

Example 14: **4-(8-Cycloheptyl-4-ethyl-7-oxo-7,8-dihydro-pyrido[2,3-d]pyrimidin-2-ylamino)-1-methyl-1H-pyrrole-2-carboxylic acid methyl ester.** The title compound was synthesized from Intermediate 11 in a manner analogous to the synthesis of Example 1 from Intermediate 9.

Example 15: **4-(8-Cycloheptyl-4-methyl-7-oxo-7,8-dihydro-pyrido[2,3-d]pyrimidin-2-ylamino)-1-methyl-1H-pyrrole-2-carboxylic acid.** The title compound from Example 1 (0.25 g. 0.6 mmol) was dissolved in ethanol (20 ml) and treated with 1N NaOH (10 ml) and allowed to stir for 18 hours. The mixture was heated to reflux for 3 hours, cooled, the solvent was removed under reduced pressure and then diluted with water. The pH of the solution was made acidic by the addition of HCl and the resulting solid was removed by filtration. The solid was washed with water and dried to afford 0.2 g of the title compound (84%).

The title compounds of Examples 16 through 28 were synthesized in a manner analogous to Example 15.

Example 16: **4-(4,8-Dimethyl-7-oxo-7,8-dihydro-pyrido[2,3-d]pyrimidin-2-ylamino)-1-(1-phenyl-ethyl)-1H-pyrrole-2-carboxylic acid.**

Example 17: **1-Methyl-4-[4-methyl-8-(3-methyl-cyclohexyl)-7-oxo-7,8-dihydro-pyrido[2,3-d]pyrimidin-2-ylamino]-1H-pyrrole-2-carboxylic acid.**

Example 18: **4-(8-Cyclopropyl-4-methyl-7-oxo-7,8-dihydro-pyrido[2,3-d]pyrimidin-2-ylamino)-1-methyl-1H-pyrrole-2-carboxylic acid.**

Example 19: **1-Methyl-4-[4-methyl-7-oxo-8-(tetrahydro-pyran-4-yl)-7,8-dihydro-pyrido[2,3-d]pyrimidin-2-ylamino]-1H-pyrrole-2-carboxylic acid.**

Example 20: **4-(8-Cycloheptyl-4-ethyl-7-oxo-7,8-dihydro-pyrido[2,3-d]pyrimidin-2-ylamino)-1-methyl-1H-pyrrole-2-carboxylic acid.**

Example 21: **4-(8-Cyclobutyl-4-methyl-7-oxo-7,8-dihydro-pyrido[2,3-d]pyrimidin-2-ylamino)-1-methyl-1H-pyrrole-2-carboxylic acid.**

Example 22: **4-(8-Cycloheptyl-4-methyl-7-oxo-7,8-dihydro-pyrido[2,3-d]pyrimidin-2-ylamino)-1-isopropyl-1H-pyrrole-2-carboxylic acid.**

Example 23: **[3-(8-Cycloheptyl-4-methyl-7-oxo-7,8-dihydro-pyrido[2,3-d]pyrimidin-2-ylamino)-pyrrol-1-yl]-acetic acid.**

The title compounds of Examples 24 through 28 were synthesized in a manner analogous to Example 15 by replacing ethoxyphosphinoyl-acetic acid ethyl ester with triethyl 2-fluoro-2-phosphonoacetate.

Example 24: **4-(8-Cyclopentyl-6-fluoro-4-methyl-7-oxo-7,8-dihydro-pyrido[2,3-d]pyrimidin-2-ylamino)-1-methyl-1H-pyrrole-2-carboxylic acid.**

Example 25: **4-[6-Fluoro-4-methyl-7-oxo-8-(tetrahydro-pyran-4-yl)-7,8-dihydro-pyrido[2,3-d]pyrimidin-2.ylamino]-1-methyl-1H-pyrrole-2-carboxylic acid.**

Example 26: **4-[6-Fluoro-4-methyl-7-oxo-8-(tetrahydro-pyran-4-ylmethyl)-7,8-dihydro-pyrido[2,3-d]pyrimidin-2-ylamino]-1-methyl-1H-pyrrole-2-carboxylic acid.**

Example 27: **4-(6-Fluoro-8-(4-methoxy-cyclohexyl)-4-methyl-7-oxo-7,8-dihydro-pyrido[2,3-d]pyrimidin-2-ylamino]-1-methyl-1H-pyrrole-2-carboxylic acid.**

Example 28: **4-(8-Cyclopentyl-6-fluoro-4-methyl-7-oxo-7,8-dihydro-pyrido[2,3-d]pyrimidin-2-ylamino)-1-(1-phenyl-ethyl)-1H-pyrrole-2-carboxylic acid.**

Example 29: **4-(6-Chloro-8-cyclopentyl-4-methyl-7-oxo-7,8-dihydro-pyrido[2,3-d]pyrimidin-2-ylamino)-1-methyl-1H-pyrrole-2-carboxylic acid methyl ester.**

To a solution of 8-cyclopentyl-4-methyl-2-methylsulfanyl-8*H*-pyrido[2,3-*d*]pyrimidin-7-one (2.5 g, 9.08 mmol) in 36 mls of DMF (N,N-dimethylformamide) was added N-chlorosuccinimide (1.81 g, 13.55 mmol). The reaction mixture was stirred for 30 hours at room temperature. The reaction mixture was poured into water and ethyl acetate. The organic layer was collected and the aqueous layer was extracted twice with ethyl acetate. The combined organic layers were washed with brine, dried over magnesium sulfate, the ethyl acetate was removed under reduced pressure to give 4.9 g of solid. The crude product was chromatographed on silica gel eluting with a gradient of 0% ethyl acetate to 40% ethyl acetate/ 60% hexane and gave the 1.00 g of 6-chloro-8-cyclopentyl-4-methyl-2-methylsulfanyl-8*H*-pyrido[2,3-*d*]pyrimidin-7-one as an orange solid. ¹H NMR (400 MHz, CDCl₃): δ ppm 1.70 (m, 2 H) 1.89 (m, 2 H) 2.10 (m, 2 H) 2.31 (m, 2 H) 2.61 (s, 3 H) 2.67 (s, 3 H) 6.06 (tt, *J*=8.91, 8.74 Hz, 1 H) 7.95 (s, 1 H). MS (M+1): 310.1.

To a solution of 6-chloro-8-cyclopentyl-4-methyl-2-methylsulfanyl-8*H-*pyrido[2,3-*d*]pyrimidin-7-one (1.00 g, 3.22 mmol) in chloroforms(20 ml) was added M-CPBA (m-chloroperoxybenzoic acid: 0.760 g, 3.39 mmol). The reaction mixture was stirred at room temperature for 35 minutes and was monitored by HPLC. The reaction was quenched with saturated sodium carbonate and the organic layer was collected. The organic layer was extracted twice with a solution of saturated sodium carbonate, and then once with brine. The mixture was then dried with magnesium sulfate and the chloroform was removed under reduced pressure to give 6-chloro-8-cyclopentyl-4-methyl-2-methanesulfinyl-8*H-*pyrido[2,3-*d*]pyrimidin-7-one (0.970 g as a faint yellow solid). ¹H NMR (400 MHz, CDCl₃): δ ppm 1.69 (m, 2 H) 1.95 (m, 2 H) 2.14 (m, 2 H) 2.24 (m, 2 H) 2.84 (s, 3 H) 2.97 (s, 3 H) 6.08 (m, 1 H) 8.07 (s, 1 H). MS (M+1): 326.1.

To a solution of 6-chloro-8-cyclopentyl-4-methyl-2-methanesulfinyl-8*H-*pyrido[2,3-*d*]pyrimidin-7-one (0.325 g, 1.00 mmol) in acetonitrile (3 ml) and DMF (5 drops) was added triethyl amine (0.29 ml, 2.09 mmol) and 4-amino-1methyl-1*H*-pyrrole-2-carboyxlic acid- methyl ester hydrochloride (0.38 g, 2.00 mmol). The reaction was heated at 120 °C for 2 days, then cooled and the acetonitrile was removed under reduced pressure to give 1 g of a crude solid. The crude product was chromatographed on silica gel eluting with a gradient of 100% hexanes to 100% ethyl acetate. To remove additional impurities the compound was taken up in ether, filtered, and dried to give 45 mg of the title compound. ¹H NMR (400 MHz, CDCl₃): δ ppm 1.64 (m, 2 H) 1.87 (m, 2 H) 2.06(m, 2H) 2.34 (m, 2 H) 2.60 (s, 3 H) 3.82 (s, 3H) 3.94 (s, 3 H) 6.00 (m, 1 H) 6.94 (m, 1 H) 7.15(m, 1H) 7.88 (s, 1 H). MS (M+1): 416.1.

**Example 30: 4-(6-Chloro-8-ethyl-4-methyl-7-oxo-7,8-dihydro-pyrido[2,3-dlpyrimidin-2-ylandno)-1-methyl-1H-pyrrole-2-carboxylic acid methyl ester.** The title compound of Example 30 was synthesized in a manner analogous to Example 29.

Example 31: **4-(6-Chloro-8-ethyl-4-methyl-7-oxo-7,8-dihydro-pyrido[2,3-d]pyrimidin-2-ylamino)-1-methyl-1H-pyrrole-2-carboxylic acid.** The title compound of Example 31 was synthesized in a manner analogous to Example 29, followed an ester hydrolysis step as carried out in Example 15.

Example 32: **4-[6-Bromo-8-(2-methoxy-ethyl)-4-methyl-7-oxo-7,8-dihydro-pyrido[2,3-d]pyrimidin-2-ylamino]-1-methyl-1H-pyrrole-2-carboxylic acid.** The title compound of Example 32 was synthesized in a manner analogous to Example 31, by replacing N-chlorosuccinimide with N-bromosuccinimide.

Example 33: **4-[6-Bromo-8-(4-methoxy-benzyl)-4-methyl-7-oxo-7,8-dihydro-pyrido[2,3-d]pyrimidin-2-ylamino]-1-methyl-1H-pyrrole-2-carboxylic acid.** The title compound of Example 33 was synthesized in a manner analogous to Example 32.

Example 34: **4-[6-(3-Benzyloxy-phenyl)-8-(2-methoxy-ethyl)-4-methyl-7-oxo-7,8-dihydro-pyrido[2,3-d]pyrimidin-2-ylamino]-1-methyl-1H-pyrrole-2-carboxylic acid methyl ester.** To a solution of 6-bromo-8-(2-methoxy-ethyl)-4-methyl-2-methylsulfanyl-8*H*-pyrido[2,3-*d*]pyrimidin-7-one (0.665 g, 1.93 mmol) in methanol (3.6 ml) and toluene (3.6 ml) was added was added tetrakis(triphenylphosphine)palladium(0) (0.089 g, 0.077 mmol) and saturated sodium carbonate (3.6 ml) and 3-benzyloxy-phenyl-boronic acid (0.485 g, 2.12 mmol). The reaction mixture was heated at 110°C for 19 hours. The crude product was chromatographed on silica gel eluting with a gradient of 0% ethyl acetate to 50% ethyl acetate/ 50% hexane and gave 0.705 g, 82% yield, of the title compound as a yellow solid. ¹H NMR (400 MHz, CDCl₃): δ ppm 2.63 (s, 3 H) 2.68 (s, 3 H) 3.39 (m, 3 H) 3.76 (t, *J*=6.20 Hz, 2 H) 4.76 (t, *J*=6.22 Hz, 2 H) 5.12 (s, 2 H) 7.01 (qd, *J*=8.06, 2.44, 0.98 Hz, 1 H) 7.27 (t, *J*=1.71 Hz, 1 H) 7.33 (m, 2 H) 7.38 (m, 3 H) 7.46 (m, 2 H) 7.83 (s, 1 H). MS (M+1): 448.1.

To a solution of 6-(3-Benzyloxy-phenyl)-8-(2-methoxy-ethyl)-4-methyl-2-methylsulfanyl-8*H*-pyrido[2,3-*d*]pyrimidin-7-one (0.705 g, 1.52 mmol) in chloroform (15 ml) was added M-CPBA (m-chloroperoxybenzoic acid: 0.375 g, 1.67 mmol). The reaction mixture was stirred at room temperature for 2.5 h and was monitored by HPLC. The reaction was quenched with saturated sodium carbonate and the organic layer was collected. The organic layer was extracted twice with a solution of saturated sodium carbonate, and then once with brine. The mixture was then dried with magnesium sulfate to give 0.666 g of product. ¹H NMR (400 MHz, CDCl₃): δ ppm 2.92 (s, 3 H) 2.92 (s, 3 H) 3.31 (s, 3 H) 3.73 (td, *J*=5.68, 1.83 Hz, 2 H) 4.75 (q, *J*=5.62 Hz, 2 H) 5.06 (s, 2 H) 7.00 (qd, *J*=8.30, 2.68,0.98 Hz, 1 H) 7.21 (m, 1 H) 7.28 (m, 2 H) 7.34 (m, 3 H) 7.40 (m, 2 H) 7.87 (s, 1 H). MS (M+1): 464.1.

To a solution of 6-(3-Benzyloxy-phenyl)-2-methanesulfinyl-8-(2-methoxyethyl)-4-methyl-8*H*-pyrido[2,3-*d*]pyrimidin-7-one (0.300 g, 0.647 mmol) in acetonitrile (2 ml) and DMF (1 ml) was added triethyl amine (0.225 ml, 1.62 mmol) and 4-amino-1methyl-1H-pyrrole-2-carboxylic acid-methyl ester hydrochloride (0.248 g, 1.3 mmol). The reaction was heated at 135 °C for 24 h then 120°C for 65 h, then cooled and the acetonitrile was removed under reduced pressure to give a crude solid. The crude product was chromatographed on silica gel with dichloromethane then 20% THF/ 80% dichloromethane to give 40 mg of the title compound as a yellow solid. ¹H NMR (400 MHz, CDCl₃) δ ppm 2.55 (s, 3 H) 3.35 (s, 3 H) 3.72 (t, *J*=6.83 Hz, 2 H) 3.77 (s, 3 H) 3.90 (s, 3 H) 4.66 (m, 2 H) 5.06 (s, 2 H) 6.74 (d, *J*=1.95 Hz, 1 H) 6.92 (qd, *J*=8.30, 2.68, 0.98 Hz, 1 H) 7.00 (m, 1 H) 7.19 (s, 2 H) 7.21 (t, *J*=1.22 Hz, 1 H) 7.27 (m, 2 H) 7.32 (m, 2 H) 7.40 (m, 1 H) 7.74 (s, 1 H). MS (M+1): 554.1.

Example 35: **4-[6-(3-Benzyloxy-phenyl)-8-(4-fluoro-benzyl)-4-methyl-7-oxo-7,8-dihydro-pyrido[2,3-d]pyrimidin-2-ylamino]-1-methyl-1H-pyrrole-2-carboxylic acid.** The title compound of Example 35 was synthesized in a manner analogous to Example 34, followed an ester hydrolysis step as carried out in Example 15.

Example 36: **4-[8-(4-Methoxy-benzyl)-4,6-dimethyl-7-oxo-7,8-dihydro-pyrido[2,3-dlpyrimidin-2-ylamino]-1-methyl-1H-pyrrole-2-carboxylic acid.** To a solution of 4-[6-bromo-8-(4-methoxy-benzyl)-4-methyl-7-oxo-7,8-dihydro-pyrido[2,3-d]pyrimidin-2-ylamino]-1-methyl-1H-pyrrole-2-carboxylic acid methyl ester (0.190 g, 0.370 mmol) in DMF (4 ml) was added copper iodide (6 mg, 0.0555 mmol), PdCl₂(PPh₃)₂ (0.019 g, 0.0278 mmol), and tetramethyl tin (0.103 ml, 0.740 mmol). The reaction mixture was heated at 100°C for 17.5 hours. The crude product was chromatographed on silica gel eluting with a gradient of 40 % ethyl acetate/ 60% hexanes to 60% ethyl acetate/ 40% hexane and gave the (0.050 g, 30% yield) of 4-[8-(4-methoxy-benzyl)-4,6-dimethyl-7-oxo-7,8-dihydro-pyrido[2,3-*d*]pyrimidin-2-ylamino]-1-methyl-1*H*-pyrrole-2-carboxylic acid methyl ester as a solid. ¹H NMR (400 MHz, CDCl₃): δ ppm 2.15 (d, *J*=1.22 Hz, 3 H) 2.53 (s, 3 H) 3.68 (s, 3 H) 3.74 (s, 3 H) 3.76 (s, 3 H) 5.50 (s, 2 H) 6.72 (s, 1 H) 6.73 (m, 2 H) 6.83 (s, 1 H) 7.26 (m, 2 H) 7.52 (d, *J*=1.22 Hz, 1 H). MS (M+1): 448.1.

To a solution of 4-[8-(4-methoxy-benzyl)-4,6-dimethyl-7-oxo-7,8-dihydro-pyrido[2,3-*d*]pyrimidin-2-ylamino]-1-methyl-1*H*-pyrrole-2-carboxylic acid methyl ester (0.049 g, 0.109 mmol) in Ethanol (15 ml) and THF (15 ml) was added a solution of 1M sodium hydroxide (0.22 ml, 0.218 mmol). The reaction mixture was refluxed for 1.5 h. The reaction mixture was then cooled to room temperature and the solvent was removed under reduced pressure. The residue was poured into water and 1M hydrochloric acid was added until a pH of 5 was reached. The reaction mixture is filtered and the filter cake washed with water. The solid was dried under vacuum to give (0.39 g, 83% yield. ¹H NMR (400 MHz, DMSO-D6): δ ppm 2.09 (s, 3 H) 2.55 (s, 3 H) 3.66 (s, 3 H) 3.70 (s, 3 H) 5.49 (s, 2 H) 6.77 (s, 1 H) 6.81 (d, *J*=8.78 Hz, 2 H) 7.11 (m, 2 H) 7.33 (m, 1 H) 7.89 (s, 1 H). MS (M-1): 432.1.

Example 38: **4-[8-Cyclohexyl-4-methyl-7-oxo-7,8-dihydro-pyrido[2,3-d]pyrimidin-2-ylamino]-1-methyl-1H-pyrrole-2-carboxylic acid.** The title compound of Example 38 was synthesized in a manner analogous to Example 15.

Intermediate 12: **4-amino-1-benzyl-1H-pyrrole-2-carboxylic ethyl ester.** A solution of ethyl, 4-nitropyrrole-2-carboxylate (4.0 g, 21.7 mmol) in DMF (70 ml) was treated portion wise with NaH (0.57 g, 23.9 mmol) and allowed to stir for a 20-minute period. The mixture was treated with benzyl bromide (3.09 ml, 26 mmol), stirred for 18 hours at ambient temperature, and the solvent removed under reduced pressure. The residue was dissolved in ethyl acetate, extracted with 1N NaOH and 1N HCl, dried over MgSO₄, and concentrated to dryness to afford 4-nitro-1-benzyl-1H-pyrrole-2-carboxylic ethyl ester (5.2 g). The crude product (2.5 g. 9 mmol) was then dissolved in THF/methanol (50 ml/50 ml) and treated with RaNi (0.5 g) at 51 p.s.i. hydrogen gas. The mixture was stirred until hydrogen gas uptake stopped, and then the RaNi catalyst was removed by filtration and the solvent removed under reduced pressure to afford the title compound, 2.2 g. ¹H NMR (400 MHz, DMSO-D6): δ ppm 1.2 (t, *J*=7.1 Hz, 3 H) 4.1 (q, *J*=7.1 Hz, 2 H) 5.4 (s, 2 H) 6.3 (s, 1 H) 6.5 (s, 1 H) 7.0 (d, *J*=6.8 Hz, 2 H) 7.2 (m, 1 H) 7.3 (d, *J*=7.6 Hz, 2 H).

**TABLE 1**

| Ex. | Mass Spec.¹ | ¹H NMR² |
|---|---|---|
| 1 | 410 | 1.5 (s, 5H) 1.6 (s, 5H) 1.8 (s, 2H) 2.4 (s, 2H) 2.5 (s, 3H) 3.7 (s, 4H) 3.8 (s, 4H) 5.6 (s, 1H) 6.2 (d, J=9.5 Hz, 1H) 7.1 (s, 1H) 7.2 (s, 1H) 7.8 (d, J=9.5 Hz, 1H) 9.9 (s, 1H) |
| 2 | 432.2 M-1=430.2 | 1.23 (t, J= 7.1 Hz), 1.74 (d, J= 7.1 Hz, 3H), 2.51 (s, 3H), 3.29 (s, 3H), 4.11-4.21 (m, 2H), 6.27 (d, J= 95 Hz, 1H), 6.46-6.52 (m, 1H), 6.89 (s, 1H), 7.22-7.35 (m, 5H), 7.60 (s, 1H), 7.89 (d, J=9.5 Hz, 1H), 10.03 (s, 1H) |
| 3 | 354 | 0.7 (s, 2H) 1.2 (s, 2H) 2.5 (s, 3H) 2.9 (s, 1H) 3.7 (s, 3H) 3.8 (s, 3H) 6.2 (d, J=9.5 Hz, 1H) 7.0 (s, 1H) 7.6 (s, 1H) 7.9 (d, J=9.5 Hz, 1H) 10.0 (s, 1H) |
| 4 | 368 | 1.7 (m, 1H) 1.9 (m, 2H) 2.1 (m, 2H) 2.5 (s, 3H) 3.1 (m, 1H) 3.7 (s, 3H) 3.8 (s, 3H) 5.9 (m, 1H) 6.2 (d, J=9.5 Hz, 1H) 7.1 (s, 1H) 7.2 (s, 1H) 7.9 (d, J=9.5 Hz, 1H) 9.9 (s, 1H) |
| 5 | 398 | 1.5 (s, 2H) 2.6 (s, 3H) 2.9 (m, 1H) 3.5 (m,2H) 3.7 (s, 3H) 3.8 (s, 3H) 4.0 (m, 2H) 5.6 (m, 1H) 5.7 (s, 1H) 6.2 (s, 2H) 7.9 (d, J=9.8 Hz, 2H) 10.0 (s, 1H) |
| 6 | 396 | 1.2 (m, 3H) 1.4 (m, 2H) 1.5 (m, 2H) 1.7 (m, 2H) 1.8 (m, 2H) 2.5 (s, 3H) 3.7 (s, 3H) 3.8 (s, 3H) 5.5 (m, 1H) 6.2 (s, 1H) 7.1 (s, 1H) 7.9 (d, J=9.5 Hz, 1H) 10.0 (s, 1H) |
| 7 | 500 | 1.2 (d, J=14.2 Hz, 3H) 1.3 - 1.6 (m, 10H) 2.4 (m, 2H) 2.5 (s, 3H) 4.1 (q, J=7.2 Hz, 2H) 5.6 (m, 3H) 6.2 (d, J=9.0 Hz, 1H) 7.0 (m, 2H) 7.0 (s, 1H) 7.2 (m, 1H) 7.3 (m, 2H) 7.4 (s, 1H) 7.9 (d, J=9.3 Hz, 1H) |
| 8 | 382.2 M-1= 380.2 | 0.02 (m, 2H) 0.41 (d, J=8.30 Hz, 2H) 0.80 (dd, J=7.81, 3.17 Hz, 1H) 1.55 (m, 2H) 2.55 (s, 3H) 3.72 (s, 3H) 3.87 (s, 3H) 4.38 (m, 2H) 6.29 (d, J=9.52 Hz, 1H) 7.01 (s, 1H) 7.34 (s, 1H) 7.94 (d, J=9.76 Hz, 1H) 10.00 (s, 1H) |
| 9 | 400 | 1.6 (s, 3H) 1.8 (s, 3H) 1.9 (s, 3H) 2.2 (m, 5H) 2.5 (s, 4H) 3.6 (m, 1H) 3.7 (s, 7H) 3.8 (s, 4H) 3.9 (s, 3H) 6.0 (s, 2H) 7.0 (s, 1H) 7.1 (s, 1H) 7.9 (d, J=10.2 Hz, 2H) 8.2 (s, 1H) 9.8 (s, 1H) 9.9 (s, 1H) |
| 10 | 430 | 1.2-1.5 (m, 5H) 2.2 (m, 1H) 2.5 (s, 3H) 3.2 (t, J=11.0 Hz, 2H) 3.7 (s, 3H) 3.8 (m, 4H) 4.3 (d, J=7.1 Hz, 2H) 7.1 (s, 1H) 7.2 (s, 1H) 8.0 (d, J=10.5 Hz, 1H) 10.0 (s, 1H) |
| 11 | 444 | 1.4 (m, 2H) 1.6 (m, 2H) 2.0 (m, 2H) 2.5 (s, 3H) 2.8 (m, 2H) 3.3 (d, J=15.1 Hz, 3H) 3.5 (m, 1H) 3.7 (s, 3H) 3.8 (s, 3H) 5.5 (m, 1H) 7.1 (d, J=13.9 Hz, 2H) 7.9 (d, J=10.2 Hz, 1H) 10.0 (s, 1H) |
| 12 | 491 | 1.2 (m, 4H) 1.5 (s, 2H) 1.7 (s, 2H) 1.9 (s, 2H) 2.1 (s, 1H) 2.2 (s, 2H) 2.5 (s, 6H) 4.2 (m, 2H) 5.6 (s, 2H) 5.9 (s, 1H) 7.1 (s, 1H) 7.3 (d, J=4.9 Hz, 2H) 7.4 (m, 2H) 7.9 (d, J=10.2 Hz, 1H) 8.4 (s, 1H) 8.4 (s, 1H) 10.0 (s, 1H) |
| 13 | 504 | 1.2 (t, J=7.1 Hz, 3H) 1.4 (s, 2H) 1.7 (d, J=10.2 Hz, 4H) 1.9 (s, 2H) 2.1 (s, 2H) 2.5 (s, 3H) 4.2 (m, 2H) 6.0 (s, 1H) 6.5 (s, 1H) 7.0 (s, 1H) 7.1 (s, 2H) 7.2 (d, J=7.3 Hz, 1H) 7.3 (s, 2H) 7.5 (s, 1H) 7.9 (d, J=10.2 Hz, 1H) 10.0 (s, 1H) |
| 14 | 424 | 1.2 (t, J=7.4 Hz, 3H) 1.6 -1.8 (m, 10H) 2.4 (s, 1H) 2.9 (q, J=7.6 Hz, 2H) 3.7 (s, 3H) 3.8 (s, 3H) 5.7 (m, 1H) 6.2 (s, 1H) 7.1 (s, 1H) 7.2 (s, 1H) 7.9 (d, J=9.5 Hz, 1H) 9.9 (s, 1H) |
| 15 | 396 | 1.5 (s, 5H) 1.6 (s, 4H) 1.7 (s, 2H) 2.4 (s, 2H) 2.5 (s, 8H) 2.6 (s, 1H) 3.3 (s, 3H) 5.6 (s, 1H) 6.2 (m, 1H) 6.9 (s, 1H) 7.2 (s, 1H) 7.8 (d, J=9.5 Hz, 1H) 9.9 (m, 1H) |
| 16 | 404.2 M-1= 402.2 | 1.74 (d, J= 7.1 Hz, 3H), 2.51 (s, 3H), 3.34 (s, 3H), 6.26 (d, J= 9.5 Hz, 1H), 6.54-6.59 (m, 1H), 6.83 (s, 1H), 7.22-7.35 (m, 5H), 7.55 (s, 1H), 7.90 (d, J=9.5 Hz, 1H), 10.00 (s, 1H), 12.21 (bs, 1H) |
| 17 | 346 | 0.9 (m, 3H) 1.5 - 1.8 (m, 5H) 2.2 (m,1H) 2.5 (s, 3H) 3.8 (s, 3H) 5.5 (m, 1H) 6.2 (d, J=9.5 Hz, 1H) 7.2 (m, 2H) 7.8 (d, J=9.5 Hz, 1H) 9.9 (s, 1H) 12.2 (s, 1H) |
| 18 | 340 | 0.7 (s, 2H) 1.2 (s, 1H) 1.3 (s, 1H) 2.5 (m, 10H) 2.9 (s, 1H) 3.8 (s, 3H) 6.2 (d, J=9.5 Hz, 1H) 7.0 (s, 1H) 7.5 (s, 1H) 7.9 (d, J=9.8 Hz, 1H) 9.9 (s, 1H) 12.1 (s, 1H) |
| 19 | 384 | 1.5 (d, J=19.5 Hz, 2H) 2.5 (s, 3H) 2.9 (m, 2H) 3.5 (t, J=12.0 Hz, 2H) 3.8 (s, 3H) 4.0 (d, J=8.5 Hz, 2H) 5.7 (m, 1H) 6.2 (d, J=9.5 Hz, 1H)6.9 (m, 2H) 7.9 (d, J=9.5 Hz, 1H) 9.9 (s, 1H) 12.2 (s, 1H) |
| 20 | 410 | 1.1 (m, 2H) 1.2(t, J=12.2 Hz, 2H) 1.5-1.8 (m, 9H) 2.6 (m, 2H) 2.9 (m, 2H)3.3(s, 3H) 5.3 (m, 1H) 6.3 (d, J=9.3 Hz, 1H) 7.4 (m, 1H) 7.6 (d, J=7.8 Hz, 1H) 8.0 (m, 1H) 10.1 (s, 1H) 13.0 (s, 1H) |
| 21 | 354 | 1.7 (m, 1H) 1.9 (m, 1H) 2.1 (m.2H) 2.6 (s, 3H) 3.1 (m, 2H) 3.8 (s, 3H) 5.9 (m.1H) 6.2 (d, J=9.5 Hz, 1H) 7.0 (s, 1H) 7.2 (s, 1H) 7.9 (d, J=9.5 Hz, 1H) 9.8 (s, 1H) 12.2 (s, 1H) |
| 22 | 424 | 1.3 (d, J=6.6 Hz, 6H) 1.5 (m, 4H) 1.6 (m, 5H) 1.7 (m, 2H) 2.4 (m, 2H) 2.5 (s, 3H) 5.4 (m, 1H) 5.6 (m, 1H) 6.1 (d, J=8.8 Hz, 1H) 7.1 (s, 1H) 7.2 (s, 1H) 7.8 (d, J=9.5 Hz, 1H) 9.9 (s, 1H) |
| 23 | 396 | 1.6 (s, 5H) 1.7 (s, 2H) 2.1 (s, 4H) 2.3 (s, 2H) 2.5 (s, 4H) 4.8 (s, 3H) 5.5 (s, 1H) 6.2 (s, 2H) 6.6 (s, 1H) 7.0 (s, 1H) 7.8 (d, J=9.5 Hz, 2H) 8.6 (s, 1H) 12.9 (s, 1H) |
| 24 | 386 | 1.6 (m, 2H) 1.8 (m, 2H) 1.9 (m, 2H) 2.2 (m, 2H) 2.5 (s, 3H) 3.8 (s, 3H) 5.9 (m, 1H) 6.9 (s, 1H) 7.1 (s, 1H) 7.9 (d, J=10.2 Hz, 1H) 9.9 (s, 1H) 12.1 (s, 1H) |
| 25 | 402 | 1.5 (d, J=13.9 Hz, 2H) 2.5 (s, 3H) 2.9 (m, 2H) 3.5 (t, J=10.2 Hz, 2H) 3.8 (s, 3H) 4.0 (m, 2H) 5.7 (m, 1H) 7.0 (s, 1H) 7.2 (s, 1H) 7.9 (d, J=10.2 Hz, 1H) 9.9 (s, 1H) 12.2 (s, 1H) |
| 26 | 416 | 1.3 (d, J=11.2 Hz, 3H) 1.4 (d, J=10.0 Hz, 3H) 2.2 (s, 2H) 2.5 (s, 6H) 3.1 (m, 4H) 3.2 (s, 1H) 3.8 (d, J=19.8 Hz, 8H) 4.2 (s, 3H) 7.0 (s, 1H) 7.2 (s, 1H) 7.9 (d, J=10.2 Hz, 1H) 9.9 (s, 1H) 12.2 (s, 1H) |
| 27 | 430 | 1.4 (d, J=7.6 Hz, 2H) 1.6 (m, 2H) 2.0 (d, J=13.9 Hz, 2H) 2.5 (s, 3H) 2.8 (m, 2H) 3.3 (s, 3H) 3.4 (s, 1H) 3.8 (s, 3H) 5.6 (m, 1H) 7.1 (m, 2H) 7.9 (d, J=10.2 Hz, 1H) 9.9 (s, 1H) |
| 28 | 476 | 1.4 (s, 2H) 1.6 (s, 1H) 1.7 (d, J=7.1 Hz, 4H) 1.9 (s, 2H) 2.2 (s, 2H) 2.5 (s, 3H) 3.3 (s, 3H) 6.0 (s, 1H) 6.6 (s, 1H) 6.9 (s, 1H) 7.1 (s, 2H) 7.2 (s, 1H) 7.3 (s, 2H) 7.5 (s, 1H) 7.9 (d, J=10.2 Hz, 1H) 9.9 (s, 1H) 12.3 (s, 1H) |
| 29 | 416.1 | 1.64 (m, 2H) 1.87 (m, 2H) 2.0 (m, 2H) 2.34 (m, 2H) 2.60 (s, 3H) 3.82 (s, 3H) 3.94 (s, 3H) 6.00 (m, 1H) 6.94 (m, 1H) 7.15(m, 1H) 7.88 (s, 1H). |
| 30 | 376.1 | 1.29 (t, J=6.95 Hz, 3H) 2.55 (s, 3H) 3.72 (s, 3H) 3.85 (s, 3H) 4.39 (d, J=7.08 Hz, 2H) 6.98 (s, 1H) 7.34 (s, 1H) 8.27 (s, 1H). |
| 31 | 362.0 M-1=360.1 | 1.28 (t, J=6.83 Hz, 3H) 2.48 (s, 3H) 3.83 (s, 3H) 4.38 (q, J=6.59 Hz, 2H) 6.93 (s, 1H) 7.30 (s, 1H) 8.26 (s, 1H) 10.10 (s, 1H) |
| 32 | 438.0 | 2.51 (s, 3H) 3.22 (s, 3H) 3.59 (t, J=6.47 Hz, 2H) 3.78 (s, 3H) 4.49 (t, J=6.10 Hz, 2H) 6.78 (s, 1H) 7.37 (s, 1H) 8.39 (s, 1H) 10.09 (s, 1H) |
| 33 | 500.0 M-1=4.98.0 | 2.57 (s, 3H) 3.66 (s, 3H) 3.70 (s, 3H) 5.52 (s, 2H) 6.79 (s, 1H) 6.82 (d, J=8.78 Hz, 2H) 7.07 (s, 1H) 7.15 (d, J=8.54 Hz, 2H) 8.49 (s, 1H) 10.08 (s, 1H) |
| 34 | 554.2 M-1=552.2 | 2.5 (s, 3H) 3.4 (s, 3H) 3.7 (m, 2H) 3.8 (s, 3H) 3.9 (s, 3H) 4.7 (m, 2H) 5.1 (s, 2H) 6.7 (d, J=2.0 Hz, 1H) 6.9 (m, 1H) 7.2 (m, 1H) 7.3 (m, 3H) 7.3 (m, 5H) 7.7 (s, 1H) |
| 35 | 590.2 M-1=588.2 | 2.59 (s, 3H) 3.67 (s, 3H) 5.09 (s, 2H) 5.55 (s, 2H) 6.75 (s, 1H) 6.95 (m, 1H) 7.04 (m, 3H) 7.27 (m, 4H) 7.34 (m, 3H) 7.41 (s, 1H) 7.43 (d, J=1.46 Hz, 1H) 8.04 (s, 1H) 9.97 (s, 1H) |
| 36 | 432.1 | 2.09 (s, 3H) 2.55 (s, 3H) 3.66 (s, 3H) 3.70 (s, 3H) 5.49 (s, 2H) 6.77 (s, 1H) 6.81 (d, J=8.78 Hz, 2H) 7.11 (m, 2H) 7.33 (m, 1H) 7.89 (s, 1H) |
| 37 | 436.1 | 1.15 (t, J=7.20 Hz, 3H) 2.50 (m, 2H) 2.57 (s, 3H) 3.70 (s, 3H) 5.54 (s, 2H) 6.76 (s, 1H) 7.01 (m,1H) 7.08 (t, J=9.03 Hz, 2H) 7.23 (m, 2H) 7.81 (s, 1H). |
| 38 | 382 | 1.2 - 1.8 (m, 10H) 2.6 (s, 3H) 3.8 (s, 5H) 5.5 (m, 3H) 6.2 (d, J=9.5 Hz, 1H) 7.0 (s, 1H) 7.1 (s, 1H) 7.8 (d, J=9.5 Hz, 1H) 9.9 (s, 1H) 12.2 (s, 1H) |

| | | |
|---|---|---|
| ¹ M+1, except where noted as M-1. ² 400 MHz, DMSO-D6, δ ppm, except for Examples 29 and 34 where it is 400 MHz, CHCl₃, δ ppm | | |

### BIOLOGICAL EXAMPLE 1

### PI3Kγ Protein Expression and Purification Protocol

Spodtera frugiperda cells, grown in ESF921 media, were co-infected with baculovirus expressing a glu-tagged p101 and baculovirus expressing an HA-tagged p110γ, at a 3:1 ratio of p101 baculovirus to p110γ baculovirus. Sf9 cells were grown to 1 x 10⁷ total cells/mL in 10L bioreactors and harvested 48-72 hours post infection. Samples of infected cells were then tested for expression of p101/p110γ PI3 kinase by immunoprecipitation and Western Blot analysis methods (see below).

To purify PI3Kγ, 4 volumes of room temperature hypotonic lysis buffer (1 mM MgCl₂, 1 mM DTT, 5 mM EGTA, 1 mM Pefabloc, 0.5 µM aprotinin, 5 µM leupeptin, 2 µM pepstatin, 5 µM E64, pH 8) per gram of cell paste, was poured onto frozen cell pellets with stirring, then lysed in a nitrogen "bomb" at 400 psi (599HC T316, Parr Instrument Co, Moline, IL). NaCl was added to 150 mM, and sodium cholate was added to 1% and mixed for another 45 minutes. The lysates were clarified by centrifugation for 25 minutes at 14,000 rpm. The lysates were then loaded over anti-glu-linked Protein-G Sepaharose beads (Covance Research Products, Richmond, CA) using 20 mL resin/50 g cell paste. The column was washed with 15 volumes of wash buffer (1 mM DTT, 0.2 mM EGTA, 1 mM Pefabloc, 0.5 µM aprotinin, 5 µM leupeptin, 2 µM pepstatin, 5 µM E64, 150 mM NaCl, 1% sodium cholate, pH 8). PI3Kγ was eluted with 6 column volumes of wash buffer that contain 100 µg/mL of a peptide that competes for binding of the glu tag. The column fractions with the eluted protein (determined by taking OD₂₈₀ readings) were collected and dialyzed in 0.2 mM EGTA, 1 mM DTT, 1 mM Pefabloc, 5 µM leupeptin, 0.5% sodium cholate, 150 mM NaCl, and 50% glycerol, pH 8. The fractions were stored at -80°C until further use.

### BIOLOGICAL EXAMPLE 2

### G Protein Subunits Expression

Spodtera frugiperda cells were coinfected with baculovirus expressing a glu-tagged G protein β₁ and baculovirus expressing a G protein β₂, at a 1:1 ratio of glu-tagged G protein β₁ baculovirus to G protein β₂ baculovirus. Sf9 cells are grown in 10 L bioreactors and harvested 48-72 hours post infection. Samples of infected cells were tested for G protein β₁/β₂ expression by Western Blot analysis, as described below. Cell lysates were homogenized and loaded onto a column of glu-tagged beads as in Biological Example 1 and competed off the column with a glu peptide and processed as described in Biological Example 1.

### BIOLOGICAL EXAMPLE 3

### Western Blot Analysis

Protein samples were run on an 8% Tris-Glycine gel and transferred to a 45 µM nitrocellulose membrane. The blots were then blocked with 5% bovine serum albumin (BSA) and 5% ovalbumin in TBST (50 mM Tris, 200 mM NaCl, 0.1% Tween 20, ph 7.4) for 1 hour at room temperature, and incubated overnight at 4°C with primary antibody diluted 1:1000 in TBST with 0.5% BSA. The primary antibodies for the p110γ, p110α, p110β, p85α, G protein β₁, and G protein γ₂ subunits were purchased from Santa Cruz Biotechnology, Inc., Santa Cruz, CA. The p101 subunit antibodies were developed at Research Genetics, Inc., Huntsville, AL based on a p101 peptide antigen.

After incubation with the primary antibody, the blots were washed in TBST and incubated for 2 hours at room temperaure with goat-anti-rabbit HRP conjugate (Bio-Rad Laboratories, Inc., Hercules, CA, product Number 170-6515), diluted 1:10,000 in TBST with 0.5% BSA. The antibodies were detected with ECL™ detection reagents (Amersham Biosciences Corp., Piscataway, New Jersey) and quantified on a Kodak ISO400F scanner.

### BIOLOGICAL EXAMPLE 4

### Immunoprecipitation

100 µL of cell paste from Biological Example 1 or 2 was thawed and lysed on ice with 400 µL of hypotonic lysis buffer (25 mM tris, 1 mM DTT, 1 mM EDTA, 1 mM Pefabloc, 5 µM leupeptin, 5 µM E-64 (Roche), 1% Nonidet P40, pH 7.5-8). The lysate was incubated for 2 hours at room temperature with glu-tagged beads (Covance Research Products, Cambridge, England, product Number AFC-115P). The beads were washed 3 times in wash buffer (20 mM Tris, pH 7.8-8, 150 mM NaCl₂, 0.5% NP40) and the protein eluted off the beads by heating in 2 times sample buffer (Invitrogen Corporation, Carlsbad, CA, product Number LC1676).

### BIOLOGICAL EXAMPLE 5

### PI3Kγ In Vitro Kinase Assay

The inhibitory properties of the compounds in Table 1 were assayed in an in vitro PI3K assay. In a 96-well polypropylene plate, each well was spotted with 2 µL of 50 times the desired final concentration of compound in DMSO. Purified recombinant p101/p110γ protein (0.03 µg; -2.7 nM) and G protein β₁/γ₂ subunits (0.09 µg; -57.7 nM) for each reaction was combined in the assay buffer (30 mM HEPES, 100 mM NaCl, 1 mM EGTA, and 1 mM DTT). ATP and [γ-³²P-ATP] (0.09 µCi) were added to this mixture so that the final ATP concentration in the reaction was 20 µM. Lipid micelles were formed by sonicating phosphatidylinositol-4,5-diphosphate (PIP₂), phosphatidylethanolamine (PE), and Na-cholate in the assay buffer for 10 minutes, adding MgCl₂ and incubating on ice for 20 minutes, for final concentrations of 25 µM PIP₂, 300 µM PE, 0.02% Na-cholate, and 10 mM MgCl₂ in the reaction. The reactions were started by adding equal volumes lipid and enzyme mixture in a total volume of 50 µL, allowed to run for 20 minutes at room temperature, and stopped with 100 µL 75 mM H₃PO₄. The lipid product was transferred to a glass fiber filter plate and washed with 75 mM H₃PO₄ several times. The presence of radioactive lipid product (PIP₃) was measured by adding Wallac Optiphase mix to each well and counting in a Wallac 1450 Trilux plate reader (PerkinElmer Life Sciences Inc., Boston, MA 02118). The IC₅₀ for each compound tested is reported in µM in the Table 2:

**TABLE 2**

| **Example** | **IC₅₀ (µM)** | **Example** | **IC₅₀ (µM)** |
|---|---|---|---|
| 1 | 0.025 | 20 | 1.595 |
| 2 | 0.063 | 21 | 0.055 |
| 3 | 0.0180 | 22 | 0.035 |
| 4 | 0.013 | 23 | 1.085 |
| 5 | 0.024 | 24 | 0.009 |
| 6 | 0.004 | 25 | 0.040 |
| 7 | 0.036 | 26 | 0.025 |
| 8 | 0.012 | 27 | 0.019 |
| 9 | 0.012 | 28 | 0.015 |
| 10 | 0.008 | 29 | 0.006 |
| 11 | 0.005 | 30 | 0.002 |
| 12 | 0.004 | 31 | 0.005 |
| 13 | 0.160 | 32 | 0.005 |
| 14 | 1.442 | 33 | 0.017 |
| 15 | 0.033 | 34 | 0.305 |
| 16 | 0.148 | 35 | 0.340 |
| 17 | 0.180 | 36 | 0.047 |
| 18 | 0.185 | 37 | 0.056 |
| 19 | 0.034 | 38 | 0.021 |

### BIOLOGICAL EXAMPLE 6

### CDK2/cyclinA In Vitro Kinase Assay

The inhibitory properties of the compounds of Examples 19, 24, and 31 were assayed in an in vitro CDK2/cyclinA assay at 10 µM of each compound in DMSO. CDK2/cyclin A (5 - 20 mU diluted in 50 mM Hepes pH 7.5, 1 mM DTT, 0.02% Brij35, 100 mM NaCl) was assayed against Histone H1 in a final volume of 25.5 µl containing 50 mM Hepes pH7.5, 1 mM DTT, 0.02% Brij35, 100 mM NaCl, Histone H1 (1 mg/ml), 10 mM magnesium acetate and 0.02 mM [³³P-γ-ATP] (500-1000 cpm/pmole) and incubated for 30 min at room temperature. Assays were stopped by addition of 5 µl of 0.5 M (3%) orthophosphoric acid and then harvested onto P81 Unifilter plates and washed with 50 mM orthophosphoric acid. The filter plates were then counted in a scintillation counter.

The results in Table 3 are expressed as percentage of controls with DMSO alone (mean of duplicates ± the standard deviation from the mean).

**TABLE 3**

| Example | % of DMSO control (mean ± S.D.) |
|---|---|
| 19 | 101 ± 6 |
| 24 | 85 ± 9 |
| 31 | 104 ± 1 |

It is understood that the examples and embodiments described herein are for illustrative purposes only and that various modifications or changes in light thereof will be suggested to persons skilled in the art and are to be included within the scope of the appended claims.

## Claims

1. A compound of Formula I: 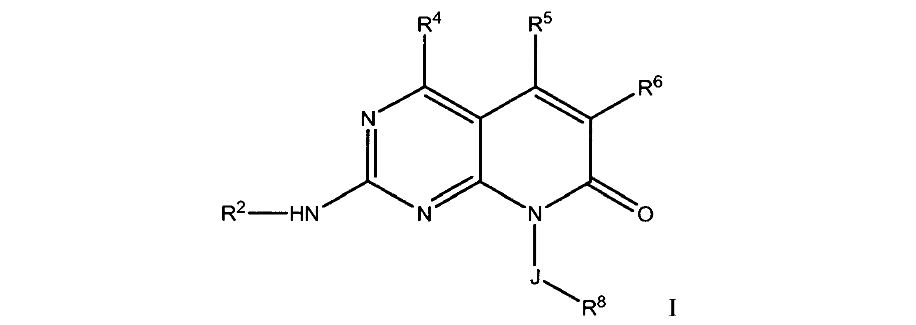 or a pharmaceutically acceptable salt thereof; wherein:
R⁴ is selected from the group consisting of:
methyl, ethyl, CF₃, CH₂F, CHF₂, and CH₂OH;
R⁵ is H or methyl;
J is absent or a C₁-C₆ alkylene;
R⁸ is selected from the group consisting of: a C₁-C₆ alkyl, a C₃-C₈ cycloalkyl, a 5- or 6-membered heterocycloalkyl, a 5- or 6- membered heteroaryl, and a phenyl;
R⁶ is selected from the group consisting of: H, halo, a phenyl, and a C₁-C₃ alkyl;
R² is: 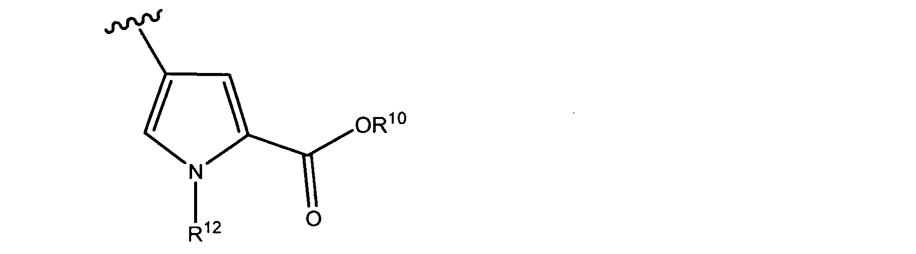
or 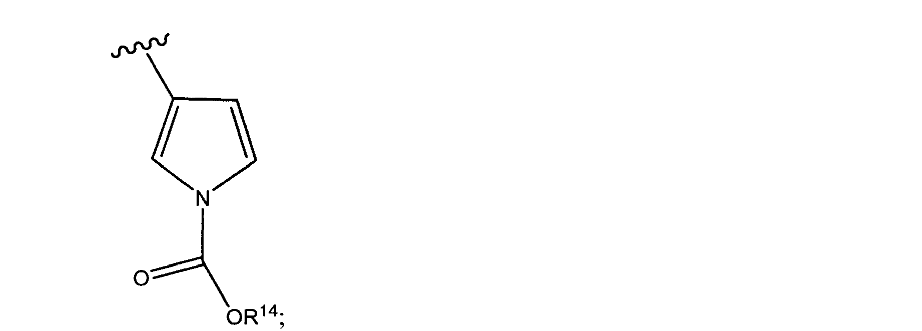
R¹⁰ is a C₁-C₆ alkyl or H;
R¹² is a C₁-C₆ alkyl, or a C₁-C₃ alkylene-R¹³,
R¹³ is a pyridinyl or a phenyl; and
R¹⁴ is a C₁-C₆ alkyl or H.

2. The compound of claim 1, wherein R⁴ is a methyl, R⁵ is H, and R² is:

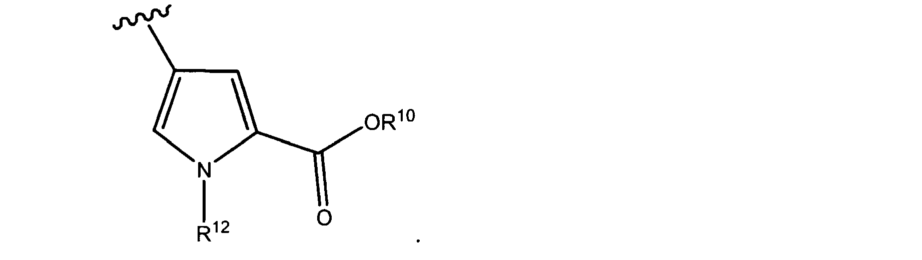

3. The compound of claim 2, wherein R¹² is a C₁-C₃alkylene-R¹³ and R⁸ is a C₁-C₆ alkyl.

4. The compound of claim 3, wherein said compound is 4-(4,8-Dimethyl-7-oxo-7,8-dihydro-pyrido[2,3-d]pyrimidin-2-ylamino)-1-(1-phenyl-ethyl)-1H-pyrrole-2-carboxylic acid.

5. The compound of claim 2, wherein R¹² is a C₁-C₃alkylene-R¹³ and R⁸ is selected from the group consisting of: a C₃-C₈ cycloalkyl, a 5- or 6- membered heterocycloalkyl, a 5- or 6-membered heteroaryl, and phenyl.

6. The compound of claim 5, wherein said compound is selected from the group consisting of:
4-(8-Cyclopentyl-6-fluoro-4-methyl-7-oxo-7,8-dihydro-pyrido[2,3-d]pyrimidin-2-ylamino)-1-pyridin-3-ylmethyl-1H-pyrrole-2-carboxylic acid ethyl ester;
4-(8-Cyclopentyl-6-fluoro-4-methyl-7-oxo-7,8-dihydro-pyrido[2,3-d]pyrimidin-2-ylamino)-1-(1-phenyl-ethyl)-1H-pyrrole-2-carboxylic acid; and
1-Benzyl-4-(8-cycloheptyl-4-methyl-7-oxo-7,8-dihydro-pyrido[2,3-d]pyrimidin-2-ylamino)-1H-pyrrole-2-carboxylic acid ethyl ester.

7. The compound of claim 2, wherein R¹² is a C₁-C₆ alkyl and R⁸ is a C₁-C₆ alkyl.

8. The compound of claim 7, wherein said compound is selected from the group consisting of:
4-(6-Chloro-8-ethyl-4-methyl-7-oxo-7,8-dihydro-pyrido[2,3-d]pyrimidin-2-ylamino)-1-methyl-1H-pyrrole-2-carboxylic acid methyl ester;
4-[6-Bromo-8-(2-methoxy-ethyl)-4-methyl-7-oxo-7,8-dihydro-pyrido[2,3-d]pyrimidin-2-ylamino]-1-methyl-1H-pyrrole-2-carboxylic acid; and
4-(6-Chloro-8-ethyl-4-methyl-7-oxo-7,8-dihydro-pyrido[2,3-d]pyrimidin-2-ylamino)-1-methyl-1H-pyrrole-2-carboxylic acid.

9. The compound of claim 2, wherein R¹² is a C₁-C₆ alkyl and R⁸ is selected from the group consisting of: a C₃-C₈ cycloalkyl, a 5- or 6-membered heterocycloalkyl, a 5- or 6-membered heteroaryl, and phenyl.

10. The compound of claim 9, wherein said compound is selected from the group consisting of:
4-(8-Cyclohexyl-4-methyl-7-oxo-7,8-dihydro-pyrido[2,3-d]pyrimidin-2-ylamino)-1-methyl-1H-pyrrole-2-carboxylic acid methyl ester;
4-[6-Fluoro-8-(4-methoxy-cyclohexyl)-4-methyl-7-oxo-7,8-dihydro-pyrido[2,3-d]pyrimidin-2-ylamino]-1-methyl-1H-pyrrole-2-carboxylic acid methyl ester;
4-[6-Fluoro-4-methyl-7-oxo-8-(tetrahydro-pyran-4-ylmethyl)-7,8-dihydro-pyrido[2,3-d]pyrimidin-2-ylamino]-1-methyl-1H-pyrrole-2-carboxylic acid methyl ester;
4-(8-Cyclopentyl-6-fluoro-4-methyl-7-oxo-7,8-dihydro-pyrido[2,3-d]pyrimidin-2-ylamino)-1-methyl-1H-pyrrole-2-carboxylic acid;
4-(8-Cyclopentyl-6-fluoro-4-methyl-7-oxo-7,8-dihydro-pyrido[2,3-d]pyrimidin-2-ylamino)-1-methyl-1H-pyrrole-2-carboxylic acid methyl ester;
4-[8-(2-Cyclopropyl-ethyl)-4-methyl-7-oxo-7,8-dihydro-pyrido[2,3-d]pyrimidin-2-ylamino]- 1-methyl-1H-pyrrole-2-carboxylic acid methyl ester;
4-(8-Cyclobutyl-4-methyl-7-oxo-7,8-dihydro-pyrido[2,3-d]pyrimidin-2-ylamino)-1-methyl-1H-pyrrole-2-carboxylic acid methyl ester;
4-[6-Bromo-8-(4-methoxy-benzyl)-4-methyl-7-oxo-7,8-dihydro-pyrido[2,3-d]pyrimidin-2-ylamino]-1-methyl-1H-pyrrole-2-carboxylic acid;
4-(8-Cyclopropyl-4-methyl-7-oxo-7,8-dihydro-pyrido[2,3-d]pyrimidin-2-ylamino)-1-methyl-1H-pyrrole-2-carboxylic acid methyl ester;
4-[6-Fluoro-8-(4-methoxy-cyclohexyl)-4-methyl-7-oxo-7,8-dihydro-pyrido[2,3-d]pyrimidin-2-ylamino]-1-methyl-1H-pyrrole-2-carboxylic acid; and
4-[8-Cyclohexyl-4-methyl-7-oxo-7,8-dihydro-pyrido[2,3-d]pyrimidin-2-ylamino]- 1-methyl-1H-pyrrole-2-carboxylic acid.

11. The compound of claim 1, wherein R⁴ is methyl, and R² is:

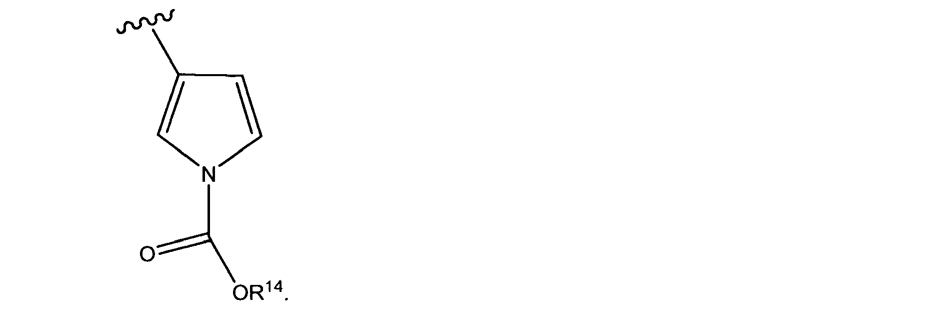

12. The compound of claim 11, wherein R⁸ is a C₁-C₆ alkyl.

13. The compound of claim 11, wherein R⁸ is selected from the group consisting of: a C₃-C₈ cycloalkyl, a 5- or 6-membered heterocycloalkyl, a 5- or 6- membered heteroaryl, and phenyl.

14. Use of a pharmaceutical composition comprising a compound of claim 1 and a pharmaceutically acceptable carrier in the manufacture of a medicament for treating a PI3K-mediated disease, wherein said PI3K-mediated disease is selected from the group consisting of:
respiratory diseases, bronchitis, asthma, chronic obstructive pulmonary disease, rheumatoid arthritis, osteoarthritis, inflammatory diseases, autoimmune diseases, cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, coronary artery disease, cancer, breast cancer, gliobastoma, endometrial carcinoma, heptocellular carcinoma, colon cancer, lung cancer, melanoma, renal cell carcinoma, thyroid carcinoma, small cell lung cancer, squamous cell lung carcinoma, glioma, breast cancer, prostate cancer, ovarian cancer, cervical cancer, leukemia, cell lymphoma, lymphoproliferative disorders, and type II diabetes.

15. Use of a compound of claim 1 in the manufacture of a medicament for the treatment of a disorder in mammals, wherein the disorder is selected from cancer, breast cancer, gliobastoma, endometrial carcinoma, heptocellular carcinoma, colon cancer, lung cancer, melanoma, renal cell carcinoma, thyroid carcinoma, small cell lung cancer, squamous cell lung carcinoma, glioma, breast cancer, prostate cancer, ovarian cancer, cervical cancer, leukemia, cell lymphoma, lymphoproliferative disorders.

16. Use of a compound of claim 1 in the manufacture of a medicament for the treatment of rheumatoid arthritis in mammals.

17. Use of a compound of claim 1 in the manufacture of a medicament for the treatment of chronic obstructive pulmonary disease in mammals.

## Patentansprüche

1. Verbindung der Formel 1: 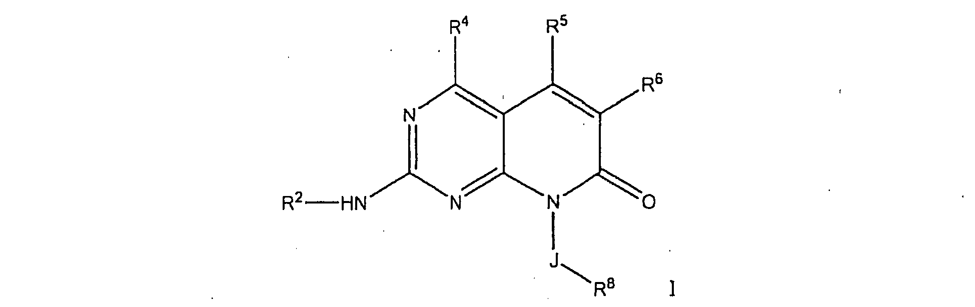 oder ein pharmazeutisch annehmbares Salz davon, wobei:
R⁴ ausgewählt ist aus der Gruppe, bestehend aus:
Methyl, Ethyl, CF₃, CH₂F, CHF₂ und CH₂OH;
R⁵ H oder Methyl ist;
J nicht vorhanden ist oder ein C₁-C₆-Alkylen ist;
R⁸ ausgewählt ist aus der Gruppe, bestehend aus: C₁-C₆-Alkyl, C₃-C₈-Cycloalkyl, einem 5- oder 6-gliedrigen Heterocycloalkyl, einem 5- oder 6-gliedrigen Heteroaryl und Phenyl;
R⁶ ausgewählt ist aus der Gruppe, bestehend aus: H, Halogen, Phenyl und C₁-C₃-Alkyl;
R² 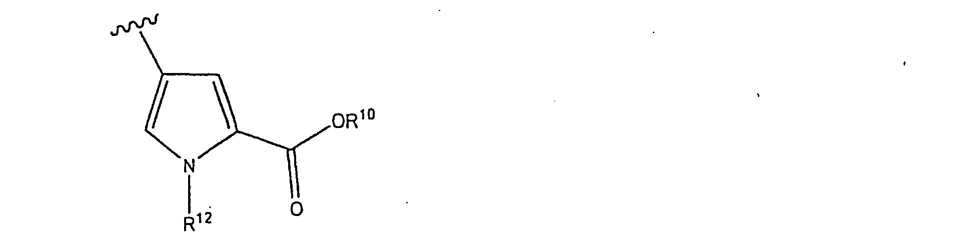 oder 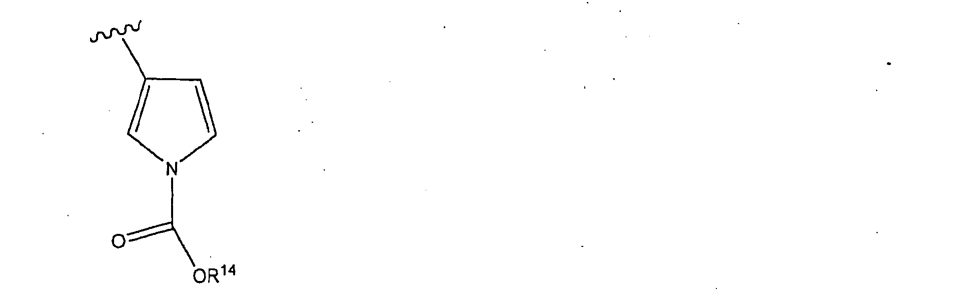 ist;
R¹⁰ C₁-C₆-Alkyl oder H ist;
R¹² C₁-C₆-Alkyl oder C₁-C₃-Alkylen-R¹³ ist,
R¹³ Pyridinyl oder Phenyl ist und
R¹⁴ C₁-C₆-Alkyl oder H ist.

2. Verbindung nach Anspruch 1, wobei R⁴ Methyl ist, R⁵ H ist und R² 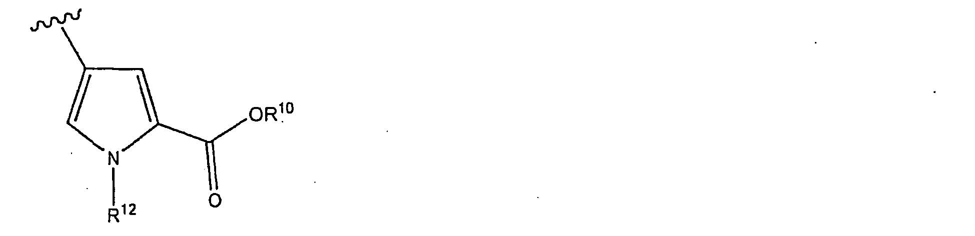 ist.

3. Verbindung nach Anspruch 2, wobei R¹² C₁-C₃-Alkylen-R¹³ ist und R⁸ C₁-C₆-Alkyl ist.

4. Verbindung nach Anspruch 3, wobei die Verbindung 4-(4,8-Dimethyl-7-oxo-7,8-dihydro-pyrido[2,3-d]pyrimidin-2-ylamino)-1-(1-phenyl-ethyl)-1H-pyrrol-2-carbonsäure ist.

5. Verbindung nach Anspruch 2, wobei R¹² C₁-C₃-Alkylen-R¹³ ist und R⁸ ausgewählt ist aus der Gruppe, bestehend aus: C₃-C₈-Cycloalkyl, einem 5- oder 6-gliedrigen Heterocycloalkyl, einem 5- oder 6-gliedrigen Heteroaryl und Phenyl.

6. Verbindung nach Anspruch 5, wobei die Verbindung ausgewählt ist aus der Gruppe, bestehend aus:
4-(8-Cyclopentyl-6-fluor-4-methyl-7-oxo-7,8-dihydro-pyrido[2,3-d]pyrimidin-2-ylamino)-1-pyridin-3-ylmethyl-1H-pyrrol-2-carbonsäureethylester;
4-(8-Cyclopentyl-6-fluor-4-methyl-7-oxo-7,8-dihydro-pyrido[2,3-d]pyrimidin-2-ylamino)-1-(1-phenyl-ethyl)-1H-pyrrol-2-carbonsäure und
1-Benzyl-4-(8-cycloheptyl-4-methyl-7-oxo-7,8-dihydro-pyrido[2,3-d]pyrimidin-2-ylamino)-1H-pyrrol-2-carbonsäure-ethylester.

7. Verbindung nach Anspruch 2, wobei R¹² C₁-C₆-Alkyl ist und R⁸ C₁-C₆-Alkyl ist.

8. Verbindung nach Anspruch 7, wobei die Verbindung ausgewählt ist aus der Gruppe, bestehend aus:
4-(6-Chlor-8-ethyl-4-methyl-7-oxo-7,8-dihydro-pyrido[2,3-d]pyrimidin-2-ylamino)-1-methyl-1H-pyrrol-2-carbonsäuremethylester;
4-[6-Brom-8-(2-methoxy-ethyl)-4-methyl-7-oxo-7,8-dihydro-pyrido[2,3-d]pyrimidin-2-ylamino]-1-methyl-1H-pyrrol-2-carbonsäure und
4-(6-Chlor-8-ethyl-4-methyl-7-oxo-7,8-dihydro-pyrido[2,3-d]pyrimidin-2-ylamino)-1-methyl-1H-pyrrol-2-carbonsäure.

9. Verbindung nach Anspruch 2, wobei R¹² C₁-C₆-Alkyl ist und R⁸ ausgewählt ist aus der Gruppe, bestehend aus: C₃-C₈-Cycloalkyl, einem 5- oder 6-gliedrigen Heterocycloalkyl, einem 5- oder 6-gliedrigen Heteroaryl und Phenyl.

10. Verbindung nach Anspruch 9, wobei die Verbindung ausgewählt ist aus der Gruppe, bestehend aus:
4-(8-Cyclohexyl-4-methyl-7-oxo-7,8-dihydro-pyrido[2,3-d]pyrimidin-2-ylamino)-1-methyl-1H-pyrrol-2-carbonsäuremethylester;
4-[6-Fluor-8-(4-methoxy-cyclohexyl)-4-methyl-7-oxo-7,8-dihydro-pyrido[2,3-d]pyrimidin-2-ylamino]-1-methyl-1H-pyrrol-2-carbonsäuremethylester;
4-[6-Fluor-4-methyl-7-oxo-8-(tetrahydro-pyran-4-ylmethyl)-7,8-dihydro-pyrido[2,3-d]pyrimidin-2-ylamino]-1-methyl-1H-pyrrol-2-carbonsäuremethylester;
4-(8-Cyclopentyl-6-fluor-4-methyl-7-oxo-7,8-dihydro-pyrido[2,3-d]pyrimidin-2-ylamino)-1-methyl-1H-pyrrol-2-carbonsäure;
4-(8-Cyclopentyl-6-fluor-4-methyl-7-oxo-7,8-dihydro-pyrido[2,3-d]pyrimidin-2-ylamino)-1-methyl-1H-pyrrol-2-carbonsäuremethylester;
4-[8-(2-Cyclopropyl-ethyl)-4-methyl-7-oxo-7,8-dihydro-pyrido[2,3-d]pyrimidin-2-ylamino]-1-methyl-1H-pyrrol-2-carbonsäuremethylester;
4-(8-Cyclobutyl-4-methyl-7-oxo-7,8-dihydro-pyrido[2,3-d]pyrimidin-2-ylamino)-1-methyl-1H-pyrrol-2-carbonsäuremethylester;
4-[6-Brom-8-(4-methoxy-benzyl)-4-methyl-7-oxo-7,8-dihydro-pyrido[2,3-d]pyrimidin-2-ylamino]-1-methyl-1H-pyrrol-2-carbonsäure;
4-(8-Cyclopropyl-4-methyl-7-oxo-7,8-dihydro-pyrido[2,3-d]pyrimidin-2-ylamino)-1-methyl-1H-pyrrol-2-carbonsäuremethylester;
4-[6-Fluor-8-(4-methoxy-cyclohexyl)-4-methyl-7-oxo-7,8-dihydro-pyrido[2,3-d]pyrimidin-2-ylamino]-1-methyl-1H-pyrrol-2-carbonsäure und
4-[8-Cyclohexyl-4-methyl-7-oxo-7,8-dihydro-pyrido[2,3-d]pyrimidin-2-ylamino]-1-methyl-1H-pyrrol-2-carbonsäure.

11. Verbindung nach Anspruch 1, wobei R⁴ Methyl ist und R² 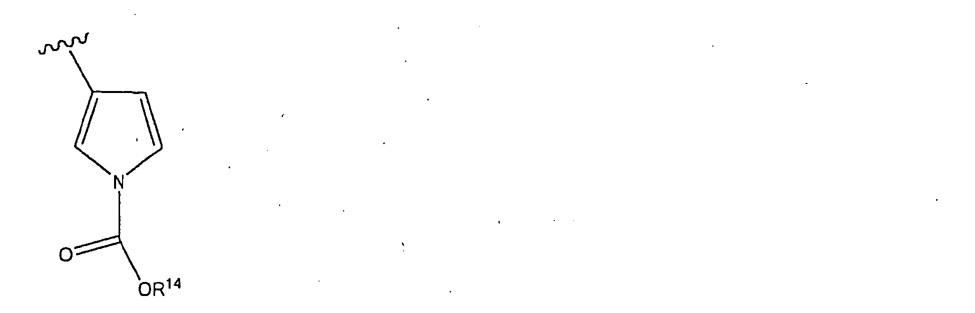 ist.

12. Verbindung nach Anspruch 11, wobei R⁸ C₁-C₆-Alkyl ist.

13. Verbindung nach Anspruch 11, wobei R⁸ ausgewählt ist aus der Gruppe, bestehend aus: C₃-C₈-Cycloalkyl, einem 5- oder 6- gliedrigen Heterocycloalkyl, einem 5- oder 6-gliedrigen Heteroaryl und Phenyl.

14. Verwendung einer pharmazeutischen Zusammensetzung, die eine Verbindung nach Anspruch 1 und einen pharmazeutisch annehmbaren Träger umfasst, bei der Herstellung eines Medikaments zur Behandlung einer PI3K-vermittelten Erkrankung, wobei die PI3K-vermittelte Erkrankung ausgewählt ist aus der Gruppe, bestehend aus: Atemwegserkrankungen, Bronchitis, Asthma, chronisch-obstruktiver Lungenerkrankung, rheumatoider Arthritis, Osteoarthritis, Entzündungserkrankungen, Autoimmunerkrankungen, kardiovaskulären Erkrankungen, Atherosklerose, Hypertonie, Thrombose der tiefen Venen, Schlaganfall, Myokardinfarkt, instabiler Angina, Thromboembolie, Lungenembolie, thrombolytischen Erkrankungen, akuter Arterienischämie, peripheren thrombotischen Okklusionen, Koronararterienerkrankung, Krebs, Brustkrebs, Glioblastom, Endometriumkarzinom, Leberzellenkarzinom, Kolonkrebs, Lungenkrebs, Melanom, Nierenzellkarzinom, Schilddrüsenkarzinom, kleinzelligem Lungenkarzinom, Plattenepithelkarzinom der Lunge, Gliom, Brustkrebs, Prostatakrebs, Eierstockkrebs, Cervixkrebs, Leukämie, Zelllymphom, lymphoproliferativen Störungen und Diabetes Typ II.

15. Verwendung einer Verbindung nach Anspruch 1 bei der Herstellung eines Medikaments für die Behandlung einer Störung bei Säugern, wobei die Störung ausgewählt ist aus Krebs, Brustkrebs, Glioblastom, Endometriumkrebs, Leberzellkarzinom, Kolonkrebs, Lungenkrebs, Melanom, Nierenzellkarzinom, Schilddrüsenkarzinom, kleinzelligem Lungenkarzinom, Plattenepithelkarzinom der Lunge, Gliom, Brustkrebs, Prostatakrebs, Eierstockkrebs, Cervixkrebs, Leukämie, Zelllymphom, lymphoproliferativen Störungen.

16. Verwendung einer Verbindung nach Anspruch 1 bei der Herstellung eines Medikaments für die Behandlung von rheumatoider Arthritis bei Säugern.

17. Verwendung einer Verbindung nach Anspruch 1 bei der Herstellung eines Medikaments für die Behandlung von chronischobstruktiver Lungenerkrankung bei Säugern.

## Revendications

1. Composé de Formole I: 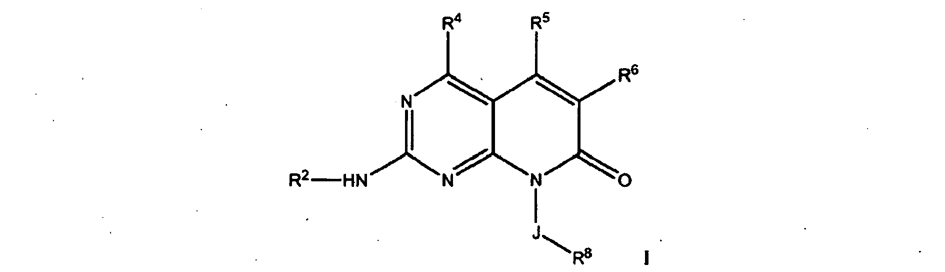 ou sel pharmaceutiquement acceptable de celui-ci, dans laquelle :
R⁴ est sélectionné dans le groupe consistant en :
méthyle, éthyle, CF₃, CH₂F, CHF₂ et CH₂OH;
R⁵ représente H ou méthyle ;
J est absent ou représente un groupe alkylène en C₁-C₆;
R⁸ est sélectionné dans le groupe consistant en :
alkyle en C₁-C₆, cycloalkyle en C₃-C₈, hétérocycloalkyle à 5 ou 6 éléments, hétéroaryle à 5 ou 6 éléments et phényle ;
R⁶ est sélectionné dans le groupe consistant en :
H, halogéno, phényle et alkyle en C₁-C₃;
R² représente : 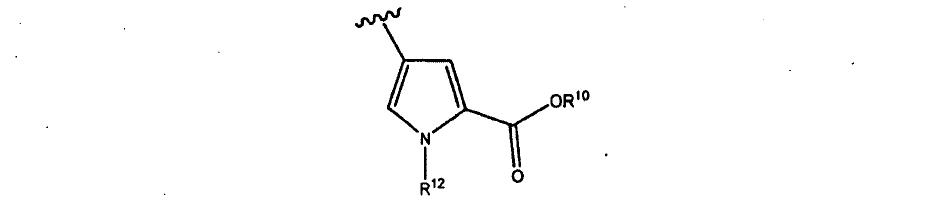
ou 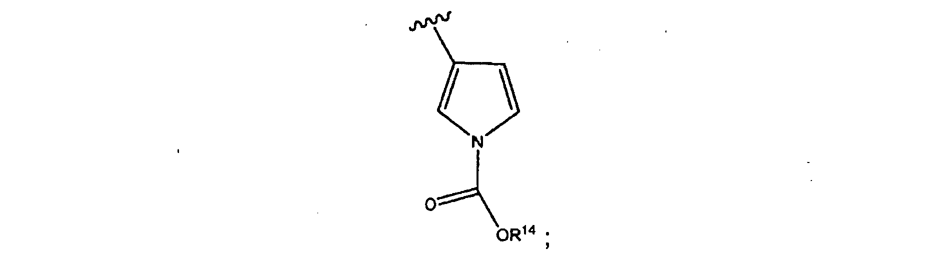 dans lesquelles :
R¹⁰ représente alkyle en C₁-C₆ ou H ;
R¹² représente alkyle en C₁-C₆ ou (alkylène en C₁-C₃)-R¹³;
R¹³ représente pyridinyle ou phényle ; et
R¹⁴ représente alkyle en C₁-C₆ ou H.

2. Composé selon la revendication 1, dans lequel R⁴ représente méthyle, R⁵ représente H et R² représente :

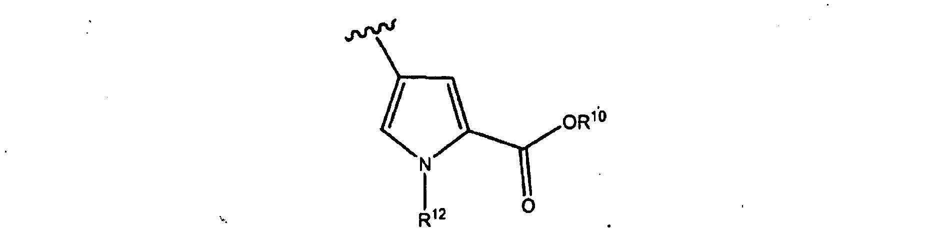

3. Composé selon la revendication 2, dans lequel R¹² représente (alkylène en C₁-C₃)-R¹³ et R⁸ représente alkyle en C₁-C₆.

4. Composé selon la revendication 3, dans lequel ledit composé est l'acide 4-(4,8-diméthyl-7-oxo-7,8-dihydro-pyrido[2,3-d]pyrimidin-2-ylamino)-1-(1-phényl-éthyl)-1H-pyrrole-2-carboxylique.

5. Composé selon la revendication 2, dans lequel R¹² représente (alkylène en C₁-C₃)-R¹³ et R⁸ est sélectionné dans le groupe consistant en : cycloalkyle en C₃-C₈, hétérocycloalkyle à 5 ou 6 éléments, hétéroaryle à 5 ou 6 éléments et phényle.

6. Composé selon la revendication 5, dans lequel ledit composé est sélectionné dans le groupe consistant en :
. l'ester éthylique de l'acide 4-(8-cyclopentyl-6-fluoro-4-méthyl-7-oxo-7,8-dihydro-pyrido[2,3-d]pyrimidin-2-ylamino)-1-pyridin-3-ylméthyl-1H-pyrrole-2-carboxylique ;
. l'acide 4-(8-cydopentyl-6-fluoro-4-méthyl-7-oxo-7,8-dihydro-pyrido[2,3-d]pyrimidin-2-ylamino)-1-(1-phényl-éthyl)-1 H-pyrrole-2-carboxylique ; et
. l'ester éthylique de l'acide 1-benzyl-4-(8-cycloheptyl-4-méthyl-7-oxo-7,8-dihydro-pyrido[2,3-d]pyrimidin-2-ylamino)-1H-pyrrole-2-carboxylique.

7. Composé selon la revendication 2, dans lequel R¹² représente alkyle en C₁-C₆ et R⁸ représente alkyle en C₁-C₆.

8. Composé selon la revendication 7, dans lequel ledit composé est sélectionné dans le groupe consistant en :
. l'ester méthylique de l'acide 4-(6-chloro-8-éthyl-4-méthyl-7-oxo-7,8-dihydro-pyrido[2,3-d]pyrimidin-2-ylamino)-1-méthyl-1H-pyrrole-2-carboxylique ;
. l'acide 4-[6-bromo-8-(2-méthoxyéthyl)-4-méthyl-7-oxo-7,8-dihydro-pyrido[2,3-d]pyrimidin-2-ylamino]-1-méthyl-1H-pyrrole-2-carboxylique ; et
. l'acide 4-(6-chloro-8-éthyl-4-méthyl-7-oxo-7,8-dihydro-pyrido[2,3-d]pyrimidin-2-ylamino)-1-méthyl-1 H-pyrrole-2-carboxylique.

9. Composé selon la revendication 2, dans lequel R¹² représente alkyle en C₁-C₆ et R⁸ est sélectionné dans le groupe consistant en : cycloalkyle en C₃-C₈, hétérocycloalkyle à 5 ou 6 éléments, hétéroaryle à 5 ou 6 éléments et phényle.

10. Composé selon la revendication 9, dans lequel ledit composé est sélectionné dans le groupe consistant en :
l'ester méthylique de l'acide 4-(8-cyclohexyl-4-méthyl-7-oxo-7,8-dihydro-pyrido[2,3-d]pyrimidin-2-ylamino)-1-méthyl-1H-pyrrole-2-carboxylique ;
l'ester méthylique de l'acide 4-[6-fluoro-8-(4-méthoxy-cyclohexyl)-4-méthyl-7-oxo-7,8-dihydro-pyrido[2,3-d]pyrimidin-2-ylamino]-1-méthyl-1 H-pyrrole-2-carboxylique ;
l'ester méthylique de l'acide 4-[6-fluoro-4-méthyl-7-oxo-8-(tétrahydro-pyran-4-ylméthyl)-7,8-dihydro-pyrido[2,3-d]pyrimidin-2-ylamino]-1-méthyl-1H-pyrrole-2-carboxylique ;
l'acide 4-(8-cyclopentyl-6-fluoro-4-méthyl-7-oxo-7,8-dihydro-pyrido[2,3-d]pyrimidin-2-ylamino)-1-méthyl-1H-pyrrole-2-carboxylique ;
l'ester méthylique de l'acide 4-(8-cyclopentyl-6-fluoro-4-méthyl-7-oxo-7,8-dihydro-pyrido[2,3-d]pyrimidin-2-ylamino)-1-méthyl-1H-pyrrole-2-carboxylique ;
l'ester méthylique de l'acide 4-[8-(2-cyclopropyl-éthyl)-4-méthyl-7-oxo-7,8-dihydro-pyrido[2,3-d]pyrimidin-2-ylamino]-1-méthyl-1H-pyrrole-2-carboxylique ;
l'ester méthylique de l'acide 4-(8-cyclobutyl-4-méthyl-7-oxo-7,8-dihydro-pyrido[2,3-d]pyrimidin-2-ylamino)-1-méthyl-1H-pyrrole-2-carboxylique ;
l'acide 4-[6-bromo-8-(4-méthoxy-benzyl)-4-méthyl-7-oxo-7,8-dihydro-pyrido[2,3-d]pyrimidin-2-ylamino]-1-méthyl-1H-pyrrole-2-carboxylique ;
l'ester méthylique de l'acide 4-(8-cyclopropyl-4-méthyl-7-oxo-7,8-dihydro-pyrido[2,3-d]pyrimidin-2-ylamino)-1-méthyl-1H-pyrrole-2-carboxylique ;
l'acide 4-[6-fluoro-8-(4-méthoxy-cyclohexyl)-4-méthyl-7-oxo-7,8-dihydro-pyrido[2,3-d]pyrimidin-2-ylamino]-1-méthyl-1 H-pyrrole-2-carboxylique ; et
l'acide 4-(8-cyclohexyl-4-méthyl-7-oxo-7,8-dihydro-pyrido[2,3-d]pyrimidin-2-ylamino)-1-méthyl-1H-pyrrole-2-carboxylique.

11. Composé selon la revendication 1, dans lequel R⁴ représente méthyle et R² représente :

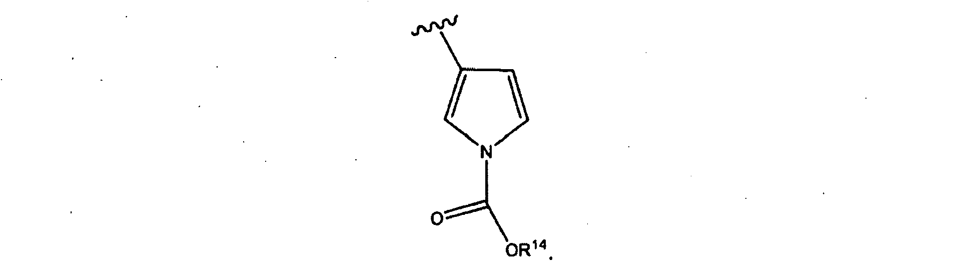

12. Composé selon la revendication 11, dans lequel R⁸ représente alkyle en C₁-C₆.

13. Composé selon la revendication 11, dans lequel R⁸ est sélectionné dans le groupe consistant en : cycloalkyle en C₃-C₈, hétérocycloalkyle à 5 ou 6 éléments, hétéroaryle à 5 ou 6 éléments et phényle.

14. Utilisation d'une composition pharmaceutique comprenant un composé selon la revendication 1, et un support pharmaceutiquement acceptable, dans la fabrication d'un médicament pour le traitement d'une maladie dans laquelle intervient la P13K, ladite maladie dans laquelle intervient la P13K étant sélectionnée dans le groupe consistant en :
les maladies respiratoires, la bronchite, l'asthme, la maladie obstructive chronique pulmonaire, l'arthrite rhumatoïde, l'ostéoarthrite, les maladies inflammatoires, les maladies auto-immunes, les maladies cardiovasculaires, l'athérosclérose, l'hypertension, la thrombose veineuse profonde, l'accident vasculaire cérébral, l'infarctus du myocarde, l'angor instable, la thromboembolie, l'embolie pulmonaire, les maladies thrombolytiques, l'ischémie artérielle aiguë, les occlusions thrombotiques périphériques, la maladie des artères coronaires, le cancer, le cancer du sein, le glioblastome, le carcinome endométrial, le carcinome hépatocellulaire, le cancer du colon, le cancer du poumon, le mélanome, le carcinome des cellules rénales, le carcinome de la thyroïde, le cancer du poumon à petites cellules, le carcinome du poumon à cellules squameuses, le gliome, le cancer du sein, le cancer de la prostate, le cancer de l'ovaire, le cancer du col de l'utérus, la leucémie, le lymphome cellulaire, les troubles lymphoprolifératifs et le diabète de type II.

15. Utilisation d'un composé selon la revendication 1, dans la fabrication d'un médicament pour le traitement d'un trouble chez les mammifères, le trouble étant sélectionné parmi le cancer, le cancer du sein, le glioblastome, le carcinome endométrial, le carcinome hépatocellulaire, le cancer du colon, le cancer du poumon, le mélanome, le carcinome des cellules rénales, le carcinome de la thyroïde, le cancer du poumon à petites cellules, le carcinome du poumon à cellules squameuses, le gliome, le cancer du sein, le cancer de la prostate, le cancer de l'ovaire; le cancer du col de l'utérus, la leucémie, le lymphome cellulaire et les troubles lymphoprolifératifs.

16. Utilisation d'un composé selon la revendication 1, dans la fabrication d'un médicament pour le traitement de l'arthrite rhumatoïde chez les mammifères.

17. Utilisation d'un composé selon la revendication 1, dans la fabrication d'un médicament pour le traitement de la maladie obstructive chronique pulmonaire chez les mammifères.
